# EUROPEAN PATENT SPECIFICATION

(11) **EP 1 368 061 B1**
(45) Date of publication and mention of the grant of the patent: **24.12.2008**
(21) Application number: 01987655.6
(22) Date of filing: 22.10.2001
(51) Int. Cl.: A61K 45/00, A61K 47/00, A01N 63/00, C12N 5/00, C12N 5/06, C12N 5/12

(54) **FUSION CELLS AND CYTOKINE COMPOSITIONS FOR TREATMENT OF DISEASE**
FUSIONSZELLEN UND ZYTOKIN-ZUSAMMENSETZUNGEN ZUR BEHANDLUNG VON KRANKHEITEN
CELLULES DE FUSION ET COMPOSITIONS CYTOKINIQUES POUR LE TRAITEMENT DE MALADIES

(30) Priority: 20.10.2000 US 242154 P
(43) Date of publication of application: 10.12.2003
(73) Proprietor: OHNO, Tsuneya, Boston, MA 02116 (US)
(72) Inventor: OHNO, Tsuneya, Boston, MA 02116 (US)
(74) Representative: Chapman, Paul Gilmour
(86) International application number: PCT/US2001/047057
(87) International publication number: WO 2002/032378

(56) References cited:
- WO-A-01/62902
- WO-A-98/46785
- DE-A1- 19 633 731
- CAO X ET AL: "THERAPY OF ESTABLISHED TUMOUR WITH A HYBRID CELLULAR VACCINE GENERATED BY USING GRANULOCYTE-MACROPHAGE COLONY-STIMULATING FACTOR GENETICALLY MODIFIED DENDRITIC CELLS" 1999, IMMUNOLOGY, BLACKWELL SCIENTIFIC PUBLICATIONS, GB, PAGE(S) 616-625 , XP002950172 ISSN: 0019-2805 * the whole document *
- WANG J ET AL: "Eliciting T cell immunity against poorly immunogenic tumors by immunization with dendritic cell-tumor fusion vaccines" 1998, JOURNAL OF IMMUNOLOGY, THE WILLIAMS AND WILKINS CO. BALTIMORE, US, PAGE(S) 5516-5524 , XP002171426 ISSN: 0022-1767 * the whole document *
- FALLARINO F ET AL: "IMPROVED EFFICACY OF DENDRITIC CELL VACCINES AND SUCCESSFUL IMMUNIZATION WITH TUMOR ANTIGEN PEPTIDE-PULSED PERIPHERAL BLOOD MONONUCLEAR CELLS BY COADMINISTRATION OF RECOMBINANT MURINE INTERLEUKIN-12" INTERNATIONAL JOURNAL OF CANCER, NEW YORK, NY, US, vol. 80, no. 2, 18 January 1999 (1999-01-18), pages 324-333, XP000867038 ISSN: 0020-7136
- LESPAGNARD, L. ET AL.: 'Dendritic cells fused with mastocytoma cells elicit therapeutic antitumor immunity' INT. J. CANCER vol. 76, 1998, pages 250 - 258, XP002951123
- GONG, J. ET AL.: 'Fusions of human ovarian carcinoma cells with autologous or allogeneic dendritic cells induce antitumor immunity' J. IMMUNOL. vol. 165, 2000, pages 1705 - 1711, XP002171429
- GONG, J. ET AL.: 'Activation of antitumor cytotoxic T lymphocytes by fusions of human dendritic cells and breast carcinoma cells' PROC. NATL. ACAD. SCI. USA vol. 97, no. 6, 14 March 2000, pages 2715 - 2718, XP002170124
- AHUJA S. ET AL.: "Human dendritic cell (DC)-based anti-infeective therapy: engineering DCs to secrete functional IFN-gamma and IL-12" J. IMMUNOLOGY, vol. 161, 1998, pages 868-876, US

## Description

### 1. INTRODUCTION

The present invention relates to methods and compositions for treating and preventing cancer and infectious disease by administering a therapeutically effective dose of fusion cells formed by fusion of autologous dendritic cells and autologous non-dendritic cells in combination with a cytokine or other molecule which stimulates a cytotoxic T cell (CTL) response and/or a humoral immune response.

### 2. BACKGROUND OF THE INVENTION

There is great interest in the development of an effective immunotherapeutic composition for treating or preventing cancer and/or infectious diseases. Success at such an immunotherapeutic approach will require the development of a composition that is both capable of eliciting a very strong immune response, and, at the same time, extremely specific for the target tumor or infected cell.

### 2.1 THE IMMUNE RESPONSE

Cells of the immune system arise from pluripotent stem cells through two main lines of differentiation, the lymphoid lineage and the myeloid lineage. The lymphoid lineage produces lymphocytes, such as T cells, B cells, and natural killer cells, while the myeloid lineage produces monocytes, macrophages, and neutrophils and other accessory cells, such as dendritic cells, platelets, and mast cells. There are two main types of T cells of the lymphoid lineage, cytotoxic T lymphocytes ("CTLs") and helper T cells which mature and undergo selection in the thymus, and are distinguished by the presence of one of two surface markers, for example, CD8 (CTLs) or CD4 (helper T cells).

Lymphocytes circulate and search for invading foreign pathogens and antigens that tend to become trapped in secondary lymphoid organs, such as the spleen and the lymph nodes. Antigens are taken up in the periphery by the antigen-presenting cells (APCs) and migrate to secondary organs. Interaction between T cells and APCs triggers several effector pathways, including activation ofB cells and antibody production as well as activation of CD8⁺ cytotoxic T lymphocytes (CD8⁺ CTLs) and stimulation of T cell production of cytokines.

CTLs then kill target cells that carry the same class I MHC molecule and the same antigen that originally induced their activation. CD8⁺ CTLs are important in resisting cancer and pathogens, as well as rejecting allografts (Terstappen et al., 1992, Blood 79:666-677).

Antigens are processed by two distinct routes depending upon whether their origin is intracellular or extracellular. Intracellular or endogenous protein antigens are presented to CD8⁺ CTLs by class I major histocompatibility complex (MHC) molecules, expressed in most cell types, including tumor cells. On the other hand, extracellular antigenic determinants are presented on the cell surface of "specialized" or "professional" APCs, such as dendritic cells and macrophages, for example, by class II MHC molecules to CD4⁺ "helper" T cells (see generally, W.E. Paul, ed., Fundamental Immunology. New York: Raven Press, 1984).

Class I and class II MHC molecules are the most polymorphic proteins known. A further degree of heterogeneity of MHC molecules is generated by the combination of class I and class II MHC molecules, known as the MHC haplotype. In humans, HLA-A, HLA-B and HLA-C, three distinct genetic loci located on a single chromosome, encode class I molecules. Because T cell receptors specifically bind complexes comprising antigenic peptides and the polymorphic portion of MHC molecules, T cells respond poorly when an MHC molecule of a different genetic type is encountered. This specificity results in the phenomenon ofMHC-restricted T cell recognition and T cell cytotoxicity.

Lymphocytes circulate in the periphery and become "primed" in the lymphoid organs on encountering the appropriate signals (Bretscher and Cohn, 1970, Science 169:1042-1049). The first signal is received through the T cell receptor after it engages antigenic peptides displayed by class I MHC molecules on the surface of APCs. The second signal is provided either by a secreted chemical signal or cytokine, such as interleukin-1 (IL-1), interferon-γ, interleukin-2 (IL-2), interleukin-4 (IL-4), interleukin-7 (IL-7), and interleukin-12 (IL-12), produced by CD4⁺ helper T cells or dendritic cells, or by a plasma-membrane-bound co-stimulatory molecule, such as B7, which is present on the antigen-presenting cell membrane and is recognized by a co-receptor on the cell surface of helper T cells, called CD28, a member of the Ig superfamily. Interferon-γ and IL-12 are associated with the helper T cell subtype known as TH₁, which promote the development of CD8⁺ T cells, and IL-4 is associated with the T helper cell subtype known as TH₂, which promote the development and activation ofB cells to produce antibodies.

In addition to antigen-specific interactions during antigen presentation, antigen non-specific adhesive mechanisms also operate. These stabilize the binding of T lymphocytes to APC. Receptor molecules on APC, such as ICAM-1/CD54, LFA-3/CD58, and B7, bind corresponding co-receptors on T cells.

Thus, helper T cells receiving both signals are activated to proliferate and to secrete a variety of interleukins. CTLs receiving both signals are activated to kill target cells. However, T cells receiving the first signal in the absence of co-stimulation become anergized, leading to tolerance (Lamb et al., 1983, J. Exp. Med. 157:1434-1447; Mueller et al., 1989, Annu. Rev. Immunol. 7:445-480; Schwartz, 1992, Cell 71:1065-1068; Mueller and Jenkins, 1995, Curr. Opin. Immunol. 7:375-381).

### 2.2 IMMUNOTHERAPY AGAINST CANCER

The cytotoxic T cell response is the most important host response for the control of growth of antigenic tumor cells (Anichimi et al., 1987, Immunol. Today 8:385-389). Studies with experimental animal tumors as well as spontaneous human tumors have demonstrated that many tumors express antigens that can induce an immune response. Some antigens are unique to the tumor, and some are found on both tumor and normal cells. Several factors influence the immunogenicity of the tumor, including, for example, the specific type of carcinogen involved, and immunocompetence of the host and the latency period (Old et al., 1962, Ann. N.Y. Acad. Sci. 101:80-106; Bartlett, 1972, J. Natl. Cancer. Inst. 49:493-504). It has been demonstrated that T cell-mediated immunity is of critical importance for rejection of virally and chemically induced tumors (Klein et al., 1960, Cancer Rés. 20:1561-1572; Tevethia et al., 1974, J. Immunol. 13:1417-1423).

Adoptive immunotherapy for tumors refers to the therapeutic approach wherein immune cells with antitumor activity are administered to a tumor-bearing host, with the objective that the cells cause the regression of an established tumor, either directly or indirectly. Immunization of hosts bearing established tumors with tumor cells or tumor antigens, as well a spontaneous tumors, has often been ineffective since the tumor may have already elicited an immunosuppressive response (Greenberg, 1987, Chapter 14, in Basic and Clinical Immunology, 6th ed., ed. by Stites, Stobo and Wells, Appleton and Lange, pp. 186-196; Bruggen, 1993). Thus, prior to immunotherapy, it had been necessary to reduce the tumor mass and deplete all the T cells in the tumor-bearing host (Greenberg et al., 1983, page 301-335, in "Basic and Clinical Tumor Immunology", ed. Herbermann RR, Martinus Nijhoff).

Animal models have been developed in which hosts bearing advanced tumors can be treated by the transfer of tumor-specific syngeneic T cells (Mulé et al., 1984, Science 225:1487-1489). Investigators at the National Cancer Institute (NCI) have used autologous reinfusion of peripheral blood lymphocytes or tumor-infiltrating lymphocytes (TIL), T cell cultures from biopsies of subcutaneous lymph nodules, to treat several human cancers (Rosenberg, S.A., U.S. Patent No. 4,690,914, issued September 1, 1987; Rosenberg et al., 1988, N. Engl. J. Med., 319:1676-1680). For example, TIL expanded *in vitro* in the presence of IL-2 have been adoptively transferred to cancer patients, resulting in tumor regression in select patients with metastatic melanoma. Melanoma TIL grown in IL-2 have been identified as CD3⁺ activated T lymphocytes, which are predominantly-CD8⁺ cells with unique *in vitro* anti-tumor properties. Many long-term melanoma TIL cultures lyse autologous tumors in a specific class I MHC- and T cell antigen receptor-dependent manner (Topalian et al., 1989, J. Immunol. 142:3714).

Application of these methods for treatment of human cancers would entail isolating a specific set of tumor-reactive lymphocytes present in a patient, expanding these cells to large numbers *in vitro,* and then putting these cells back into the host by multiple infusions. Since T cells expanded in the presence of IL-2 are dependent upon IL-2 for survival, infusion of IL-2 after cell transfer prolongs the survival and augments the therapeutic efficacy of cultured T cells (Rosenberg et al., 1987, N. Engl. J. Med. 316:889-897). However, the toxicity of the high-dose IL-2 and activated lymphocyte treatment has been considerable, including high fevers, hypotension, damage to the endothelial wall due to capillary leak syndrome, and various adverse cardiac events such as arrhythmia and myocardial infarction (Rosenberg et al., 1988, N. Engl. J. Med. 319:1676-1680). Furthermore, the demanding technical expertise required to generate TILs, the quantity of material needed, and the severe adverse side effects limit the use of these techniques to specialized treatment centers.

CTLs specific for class I MHC-peptide complexes could be used in treatment of cancer and viral infections, and ways have been sought to generate them *in vitro* without the requirement for priming *in vivo*. These include the use of dendritic cells pulsed with appropriate antigens (Inaba et al., 1987, J. Exp. Med. 166:182-194; Macatonia et al., 1989, J. Exp. Med. 169:1255-1264; De Bruijn et al., 1992, Eur. J. Immunol. 22:3013-3020). RMA-S cells (mutant cells expressing high numbers of 'empty' cell surface class I MHC molecules) loaded with peptide (De Bruijn et al., 1991, Eur. J. Immunol. 21:2963-2970; De Bruijn et al., 1992, *supra*; Houbiers et al., 1993, Eur. J. Immunol. 26:2072-2077) and macrophage phagocytosed-peptide loaded beads (De Bruijn et al., 1995, Eur. J. Immunol. 25, 1274-1285).

Fusion ofB cells or dendritic cells with tumor cells has been previously demonstrated to elicit anti-tumor immune responses in animal models (Guo et al., 1994, Science, 263:518-520; Stuhler and Walden, 1994, Cancer Immunol. Immuntother. 1994, 39:342-345; Gong et al., 1997, Nat. Med. 3:558-561; Celluzzi, 1998, J. Immunol. 160:3081-3085; Gong, PCT publication WO 98/46785, dated October 23, 1998). In particular, immunization with hybrids of tumor cells and antigen presenting cells has been shown to result in protective immunity in various rodent models.

However, the current treatments, while stimulating protective immunity, do not always effectively treat a patient who already has an established disease, namely, the administration of fusion cells to a subject with a disease, does not always stimulate an immune response sufficient to eliminate the disease. Thus, a need exists for a therapeutic composition which can be used to treat, *e.g.,* cause the regression of an existing disease, *e*.*g*., cancer or infectious disease, in a patient.

WO 98/46785 to Gong et al. describes immunostimulatory compositions that contain fused cells formed by fusion between dendritic cells and non-dendritic cells, methods of using these compositions, and methods of generating dendritic cell hybrids. WO 98/46785 teaches that immature dendritic cells are more amenable to fusion than the more mature dendritic cells found in the spleen (p. 7, *ll*. 32-34).

Cao et al. (1999, Immunology 97:616-625) describes the use of fusion cells generated from splenic dendritic cells from C57BL/6 mice fused with B 16 melanoma cells as a vaccine against melanoma in mice.

Wang et al. (1998, J. Immunology 161:5516-5524) provides an example for the use of fusion cells obtained by fusing dendritic cells with syngeneic B16 melanoma cells or RMA-S lymphoma cells.

WO-A-0162902 to Lothar Kanz relates to methods and compositions for treating and preventing cancer and infectious disease using hybrid cells formed by fusion of allogeneic dendritic cells and autologous non-dendritic ceclls.

DE-A-19633731 to Johann Hinrich Peters relates to hybrid cells obtained by fusing of antigen-presenting cells with tumor cells.

Citation or discussion of a reference herein shall not be construed as an admission that such is prior art to the present invention.

### 3. SUMMARY OF THE INVENTION

The present invention relates to the use of a therapeutically effective amount of a fusion cell or a composition comprising fusion cells, wherein said fusion cells are formed by the fusion of an autologous non-dendritic tumor cell and a dendritic cell which has the same class I MHC haplotype as a mammal to be treated in combination with a IL-12 which stimulates a cytotoxic T cell response for the manufacture of a medicament for treating or preventing cancer.

Further embodiments are defined in the appended claims.

In an embodiment, the cancer is selected from the group consisting of renal cell carcinoma, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, leukemias, acute lymphocytic leukemia, acute myelocytic leukemia; chronic leukemia, polycythemia vera, lymphoma, multiple myeloma, Waldenström's macroglobulinemia, and heavy chain disease.

Herein described is a method for making a fusion with a dendritic cell and a non-dendritic comprising: (a) subjecting a population of autologous dendritic cells and a population of autologous non-dendritic cells obtained from a mammal to conditions that promote cell fusion, and (b) inactivating the population of fusion cells. In another embodiment, the cell fusion is accomplished by electrofusion. Inactivating the population of fusion cells is accomplished by γ irradiating the cells. Further described is a method for making a fusion of a human dendritic cell and a non-dendritic cell autologous to the dendritic cell. The non-dendritic cell may either be freshly isolated from a subject or alternatively obtained from a primary cell culture or from an established cell line.

As used herein, a compound, such as a cytokine, is said to be "co-administered" or in "combination" with another compound, such as a fusion cell, when either the physiological effects of both compounds, or the elevated serum concentration of both compounds can be measured simultaneously. With compounds that increase the level of endogenous production, the serum concentration of the endogenously produced cytokine and the other administered agent (*i*.*e*., fusion cell), can also be measured simultaneously when "co-administered" or in "combination". Thus, compounds may be administered either simultaneously, as separate or mixed compositions, or they may be administered sequentially provided that an elevation of their levels in serum can be measured simultaneously at some point during administration.

Unless otherwise stated the terms "combination therapy" and "combination treatments" are used herein to describe a therapeutic regimen involving co-administration of the subject fusion cells and a molecule which stimulates a CTL response and/or humoral immune response, which results in a decrease in a disease state. Reduction of a disease state can be measured, for example, by demonstration of a reduction of tumor mass, a reduction in the number of tumor cells, or a reduction of viral load in a patient infected with hepatitis or human immunodeficiency virus, in a patient.

Described is also a kit comprising, in one or more containers, a sample containing a population of dendritic cells and instructions for its use in treating or preventing cancer or an infectious disease. The kit further comprising a cuvette suitable for electrofusion.

### 4. BRIEF DESCRIPTION OF THE FIGURES

Figures 1A-C. FACS analysis of FCs. (A) DCs were stained by FITC-labeled anti-CD 80 antibody. A total of 34% of DCs were stained with anti-CD80 monoclonal antibody. (B) PKH26 was incorporated into glioma cells. More than 95% of glioma cells were positive for PKH26. (C) After incorporation of PKH26 into glioma cells, DCs and glioma cells were fused. DCs were stained with FITC-labeled anti-CD80 monoclonal antibody. A total of 39.9% of cells were positive for both PKH26 and CD80, suggesting that most DCs were fused with glioma cells.

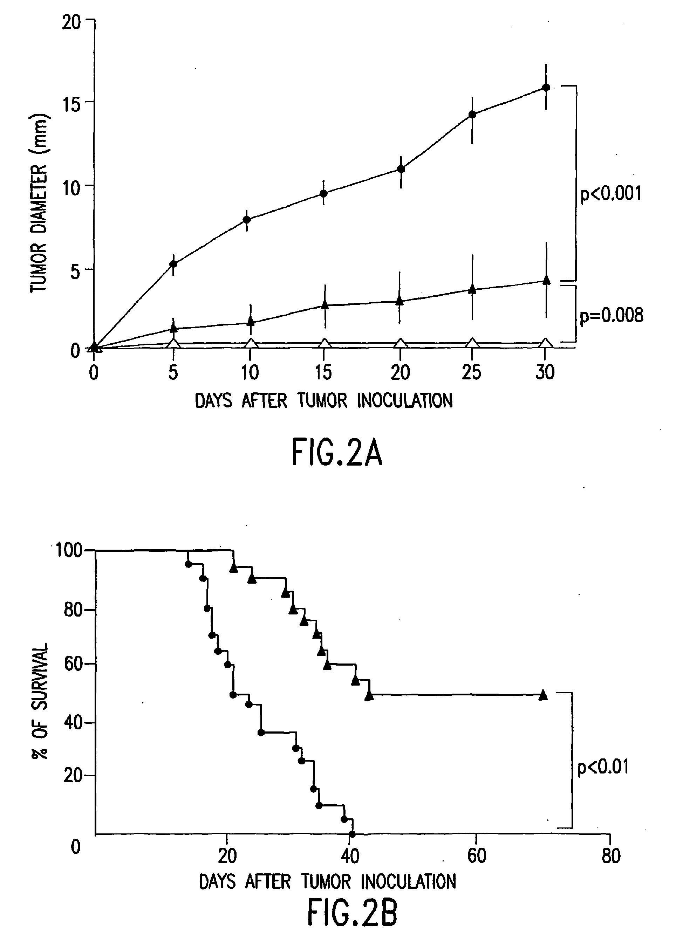

Figures 2A-B. Antitumor effects of immunization with FCs. (A) FCs (▲), DCs (▲), or irradiated parental cells as a control (●) were injected into syngeneic mice subcutaneously on days 0 and 7 (n=11 in each group). On day 14,1 x 106 parental cells were subcutaneously inoculated into the flank. The inoculated tumor cells caused large tumors within two weeks in all mice injected with irradiated parental cells. In contrast, none of the mice immunized with FCs died within six weeks. Whereas six of 11 mice immunized with DCs developed a palpable tumor that subsequently grew, none of 11 mice immunized with FCs developed a palpable tumor. (B) After immunization with FCs on days 0 and 7,1 x 10⁴ tumor cells were stereotactically inoculated into the right frontal lobe of the brain (day 14). Half of the mice immunized with FCs survived longer than 70 days (▲; n =20 in each group; p <0.001) (Fig. 2-B). All control mice died within 6 weeks (●).

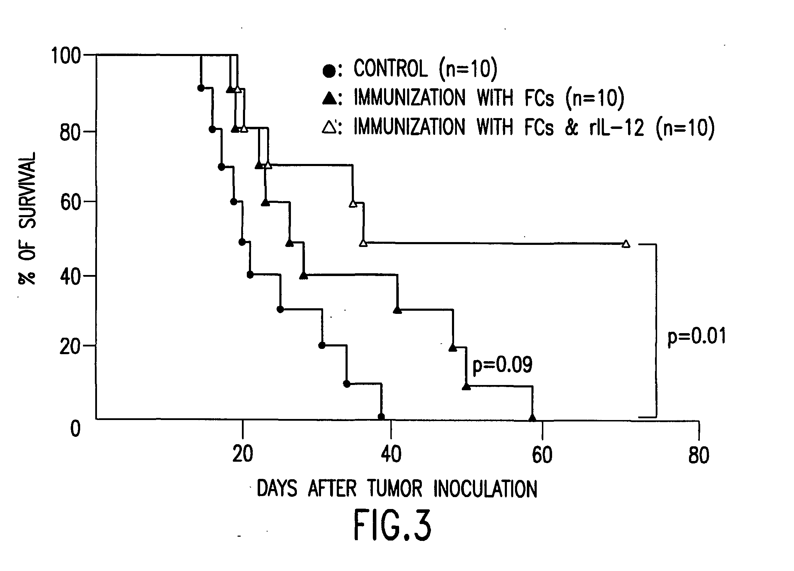

Figure 3. Survival of mice following treatment with FCs and rIL-12. Parental cells (1x10⁴) were stereotactically inoculated into the right frontal lobe (day 0). On days 5 and 12, 3 x 10⁵ FCs were subcutaneously inoculated. Several mice were given an intraperitoneal (i.p.) injection of 0.5 pg/100 µl of rmIL-12, or 100 µl of saline, every other day for two weeks (3.5 pg/mouse total) starting on Day 5 and observed for 70 days. While vaccination with FCs alone did not prolong the survival of tumor-bearing mice (▲; p > 0.05), vaccination with both FCs and rIL-12 prolonged the survival compared with the control (Δ; p = 0.01). Five of ten mice treated with FCs and rIL-1 2 survived over seventy days.

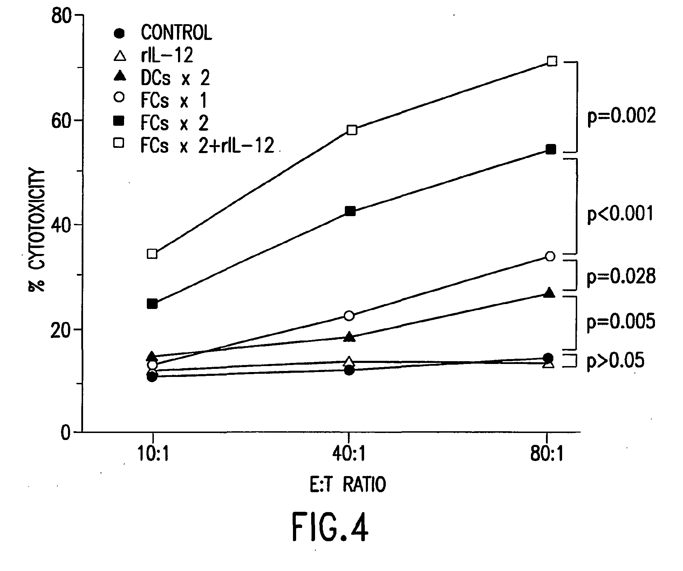

Figure 4. Cytotoxicity of spleen cells from tumor-bearing mice. SPCs were separated from untreated mice (●), mice injected with rIL-12 alone (Δ), mice injected DCs twice (days 0 and 7; ▲), mice immunized with FCs once (day 0; ○) or twice (days 0 and 7;■) and mice immunized with rIL-12 and FCs twice (days 0 and 7□;) on day 28. CTL activity on tumor cells from immunized mice, especially mice injected with rIL-12 and immunized with FCs twice, was considerably increased compared with the control and others. Antitumor activity on Yac-1 cell from treated mice increased but not considerably compared with the control (data not shown).

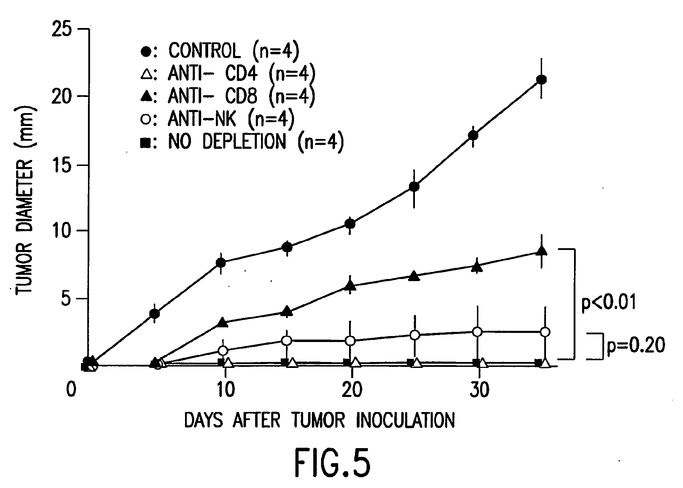

Figure 5. Regression of established subcutaneous tumors following vaccination with FCs and depletion of T-cell subsets. Lymphocyte subsets were depleted by administering anti-CD4 (Δ), anti-CD8 (▲), anti-asialo GM1 (○), or control rat IgG (■) into mice given injections of glioma cells and FCs. On days 0 and 7, FCs were subcutaneously inoculated into the flank. Subsequently parental cells were inoculated into the opposite flank on day 14. The mAbs were injected i.p. on days 7, 10, 14, and 17. The antitumor effect was reduced in mice depleted of CD8⁺ T cells (▲) (n =4 in each group). The protection conferred by FCs was not abolished by CD4⁺ T and NK cell depletion. Control mice were not vaccinated with FCs (○). Data represent means + SD.
Figures 6A-D. Immunofluorescence analysis of the developed brain tumors. A few CD4⁺ and CD8⁺ T cells were present in the tumors of non-vaccinated mice (Figures 6A, B). In contrast, many CD4⁺ and CD8⁺ T cells were seen in the tumors of vaccinated mice (Figures 6C, D). The numbers of infiltrating CD4⁺ and CD8⁺ T cells were almost the same. SR-B10.A cells were positive for GFAP.
Figure 7. Fused cells stained with both FITC (green) and PKH-26 (red) among the PEG-treated cells
Figure 8. FACS analysis, cells stained with both PKH-2GL and PKH-26, which were considered to be fusions of DCs and BNL cells, are shown in upper area of cell scattergram with high forward scatter and high side scatter. The cell fraction of high and moderate forward scatter and low side scatter contained many non-fused BNL cells, which those of low forward scatter and low side scatter contained non-fused DCs and non-fused BNL cells. About 30% of the nonadherent cells were fusions as judged from the width of area of double positive cells occupying in the whole scattergram.
Figure 9. FACS analysis of the cell fractions positive for both PKH-2GL and PKH-26 gated on scattergram and examined for antigen expression. I-A^{d}/I-E^{d} (MCH class II), CD80, CD86 and CD54 molecules, which are found on DCs, were expressed by the fusions
Figure 10. Scanning Electron Microscopy of BNL cells expressing short processes on a plain cell surface, whereas DCs have many long dendritic processes. The nonadherent fusion cells are large and ovoid with short dendritic processes.
Figure 11. Vaccination of mice with DC/BNL fusions resulted in the rejection of a challenge with BNL cells inoculated in BALB/c mice. By contrast, injection of only DCs or only irradiated BNL cells failed to prevent the development and growth of tumors.
Figure 12. Chromium-51 release assay of CTL. The effect of treatment with DC/BNL fusion cells alone against BNL tumor was not significant. However, injection of DC/BNL fusions followed by administration of IL-12 elicited a significant antitumor effect.
Figure 13. Significant cytolytic activity against BNL cells was observed using splenocytes derived from mice treated with DC/BNL fusions. The solid bars are the BNL-cells and the hatched bars are the C26-cells.
Figure 14. Splenocytes from mice treated with DC/BNL fusions in combination with IL-12 showed greater cytolytic activity against BNL cells than those treated with DC/BNL fusions alone.
Figure 15. Lytic activity of the splenocytes treated with antibody against CD4 was significantly reduced, while those treated with antibody against CD8 exhibited almost the same lytic activity as those treated with an isotype identical antibody, rat IgG₂ₐ.

### 5. DETAILED DESCRIPTION OF THE INVENTION

The invention provides uses and compositions for therapeutic applications against cancer, produced by fusion of autologous dendritic cells with autologous non-dendritic cells as defined in the appended claims. Subsequently, the fused cells are administered to a subject in need thereof, in combination with a therapeutically effective dose of a molecule which stimulates a cytotoxic T-lymphocyte response (CTL).

As described herein, autologous dendritic cells can be fused to a non-dendritic cell containing an antigen of interest, such as a cancer antigen. The resulting hybrids of dendritic cells and non-dendritic cells can be used as a potent composition against a disease condition involving an antigen, such as a cancer or an infectious disease. This approach is particularly advantageous when a specific antigen is not readily identifiable, as in the case of many cancers. For treatment of human cancer, for example, non-dendritic cells can be obtained directly from the tumor of a patient. Fusion cell compositions prepared in this way are highly specific for the individual tumor being treated.

Described below, are compositions and methods relating to such immunotherapeutic compositions. In particular, Sections 5.1, 5.2, and 5.3 describe the non-dendritic, dendritic, and the fusion cells, respectively, that are used with in the invention, and methods for their isolation, preparation, and/or generation. Target cancers and infectious diseases that can be treated or prevented using such compositions are described below in Sections 5.4 and 5.5. Section 5.6 describes the methods and use of these fusion cells as therapeutic compositions against cancer and infectious disease.

### 5.1 NON-DENDRITIC CELLS

A-non-dendritic cell of the present invention can be any tumor cell bearing an antigen of interest for use in a fusion cell-cytokine composition. Such non-dendritic cells may be isolated from a variety of desired subjects, such as a tumor of a cancer patient or a subject infected with an infectious disease. The non-dendritic cells may also be from an established cell line or a primary cell culture. The methods for isolation and preparation of the non-dendritic cells are described in detail hereinbelow.

The source of the non-dendritic cells may be selected, depending on the nature of the tumor with which the antigen is associated. The non-dendritic cells are autologous to the subject being treated, *i*.*e*., the cells used are obtained from cells of the ultimate target cells *in vivo* (*e*.*g*., of the tumor cells of the intended recipient that it is desired to inhibit). In this way, since whole cancer cells or other non-dendritic cells may be used in the present methods, it is not necessary to isolate or characterize or even know the identities of these antigens prior to performing the present methods. However, any non-dendritic tumor cell can be used as long as at least one antigen present on the cell is an antigen specific to the the target cells, and as long as the non-dendritic cell has the same class I MHC haplotype as the mammal being treated.

The non-dendritic cell is a cancer cell. Fusion cells are described that express antigens expressed by cancer cells, *e.g.*, tumor-specific antigens and tumor associated antigens, and are capable of eliciting an immune response against such cancer cells. Any tissues, or cells isolated from a cancer, including cancer that has metastasized to multiple sites, can be used for the preparation of non-dendritic cells. For example, leukemic cells circulating in blood, lymph or other body fluids can also be used, solid tumor tissue (*e.g.*, primary tissue from a biopsy) can be used. Examples of cancers that are amenable to the methods of the invention are listed in Section 5.5, 5.6, infra.

The tumor cells may be cultured to select for tumor cells to be fused with dendritic cells and prevent or limit contamination of cells to be fused with healthy, non-cancerous or uninfected cells.

The non-dendritic cells may be isolated from a tumor that is surgically removed from mammal to be the recipient of the hybrid cell compositions. Prior to use, solid cancer tissue or aggregated cancer cells should be dispersed, preferably mechanically, into a single cell suspension by standard techniques. Enzymes, such as but not limited to, collagenase and DNase may also be used to disperse cancer cells. The non-dendritic cells may be obtained from primary cell cultures, *i.e.,* cultures of original cells obtained from the body. Typically, approximately 1x10⁶ to 1x10⁹ non-dendritic cells are used for formation of fusion cells.

Approximately 1 x 10⁶ to 1 x 10⁹ non-dendritic cell are used for formation of fusion cells. Also 5 x 10⁷ to 2 x 10⁸ or 5 x 10⁷ cells may be used.

In order to prepare suitable non-dendritic cells that are cancer cells, noncancerous cells, preferably of the same cell type as the cancer desired to be inhibited can be isolated from the recipient or, less preferably, other individual who shares at least one MHC allele with the intended recipient, and treated with agents that cause the particular or a similar cancer or a transformed state; such agents may include but not limited to, radiation, chemical carcinogens, and viruses. Standard techniques can be used to treat the cells and propagate the cancer or transformed cells so produced.

For the treatment and prevention of infectious disease, it is described that an antigen having the antigenicity of a pathogen, in particular, an intracellular pathogen, such as a virus, bacterium, parasite, or protozoan, can be used. For example, a cell that is infected with a pathogen is used. A cell that is recombinantly engineered to express an antigen having the antigenicity of the pathogen may also be used. used. An exemplary list of infectious diseases that can be treated or prevented by the methods of the invention is provided in Section 5.6, below.

Alternatively, if the gene encoding a tumor-specific antigen, tumor-associated antigen or antigen of the pathogen is available, normal cells of the appropriate cell type from the intended recipient. Optionally, more than one such antigen may be expressed in the recipient's cell in this fashion, as will be appreciated by those skilled in the art, any techniques known, such as those described in Ausubel et al. (eds., 1989, Current Protocols in Molecular Biology, Greene Publishing Associates and Wiley Interscience, New York), may be used to perform the transformation or transfection and subsequent recombinant expression of the antigen gene in recipient's cells. These non-dendritic cells bearing one or more MHC molecules in common with the recipient are suitable for use in the methods for formation of fusion cells of the invention.

The non-dendritic cells used for the generation of fusion cells and the target tumor or pathogen infected cell must have at least one common MHC allele in order to elicit an immune response in the mammal. In the use of this invention the non-dendritric cells are derived from the intended recipient (*i.e.*, are autologous). Described is also that, the non-dendritic cells are nonautologous, but share at least one MHC allele with the cancer cells of the recipient. If the non-dendritic cells are obtained from the same or syngeneic individual, such cells will all have the same class I MHC haplotype. If they are not all obtained from the same subject, the MHC haplotype can be determined by standard HLA typing techniques well known in the art, such as serological tests and DNA analysis of the MHC loci. An MHC haplotype determination does not need to be undertaken prior to carrying out the procedure for generation of the fusion cells of the invention.

Non-dendritic cells, such as cells containing an antigen having the antigenicity of a cancer cell or an infectious disease cell, can be identified and isolated by any method known in the art. For example, cancer or infected cells can be identified by morphology, enzyme assays, proliferation assays, or the presence of cancer-causing viruses. If the characteristics of the antigen of interest are known, non-dendritic cells can also be identified or isolated by any biochemical or immunological methods known in the art. For example, cancer cells or infected cells can be isolated by surgery, endoscopy, other biopsy techniques, affinity chromatography, and fluorescence activated cell sorting (*e*.*g*., with fluorescently tagged antibody against an antigen expressed by the cells).

There is no requirement that a clonal or homogeneous or purified population of non-dendritic cells be used. A mixture of cells can be used provided that a substantial number of cells in the mixture contain the antigen or antigens present on the tumor cells being targeted. In a specific embodiment, the non-dendritic cells and/or dendritic cells are purified.

### 5.2 DENDRITIC CELLS

Dendritic cells can be isolated or generated from blood or bone marrow, or secondary lymphoid organs of the subject, such as but not limited to spleen, lymph nodes, tonsils, Peyer's patch of the intestine, and bone marrow, by any of the methods known in the art. Preferably, DCs used in the methods of the invention are (or terminally differentiated) dendritic cells. The source of dendritic cells is preferably human blood monocytes.

Immune cells obtained from such sources typically comprise predominantly recirculating lymphocytes and macrophages at various stages of differentiation and maturation. Dendritic cell preparations can be enriched by standard techniques (see e.g., Current Protocols in Immunology, 7.32.1-7.32.16, John Wiley and Sons, Inc., 1997). In one embodiment, for example, DCs may be enriched by depletion of T cells and adherent cells, followed by density gradient centrifugation. DCs may optionally be further purified by sorting of fuorescence-labeled cells, or by using anti-CD83 MAb magnetic beads.

Alternatively, a high yield of a relatively homogenous population of DCs can be obtained by treating DC progenitors present in blood samples or bone marrow with cytokines, such as granulocyte-macrophage colony stimulating factor (GM-CSF) and interleukin 4 (IL-4). Under such conditions, monocytes differentiate into dendritic cells without cell proliferation. Further treatment with agents such as TNFα stimulates terminal differentiation of DCs.

By way of example but not limitation, dendritic cells can be obtained from blood monocytes as follows: peripheral blood monocytes are obtained by standard methods (see, *e*.*g*., Sallusto et al., 1994, J. Exp. Med. 179:1109-1118). Leukocytes from healthy blood donors are collected by leukapheresis pack or buffy coat preparation using Ficoll-Paque density gradient centrifugation and plastic adherence. If mature DCs were desired, the following protocol may be used to culture DCs. Cells are allowed to adhere to plastic dishes for 4 hours at 37°C. Nonadherent cells are removed and adherent monocytes are cultured for 7 days in culture media containing 0.1µg/ml granulocyte-monocyte colony stimulating factor and 0.05µg/ml interleukin-4. In order to prepare dendritic cells, tumor necrosis factor-α is added on day 5, and cells are collected on day 7.

Dendritic cells obtained in this way characteristically express the cell surface marker CD83. In addition, such cells characteristically express high levels of MHC class II molecules, as well as cell surface markers CD1α, CD40, CD86, CD54, and CD80, but lose expression of CD14. Other cell surface markers characteristically include the T cell markers CD2 and CD5, the B cell marker CD7 and the myeloid cell markers CD13, CD32 (FcγR II), CD33, CD36, and CD63, as well as a large number of leukocyte-associated antigens

Optionally, standard techniques such as morphological observation and immunochemical staining, can be used to verify the presence of dendritic cells. For example, the purity of dendritic cells can be assessed by flow cytometry using fluorochrome-labeled antibodies directed against one or more of the characteristic cell surface markers noted above, *e.g.,* CD83, HLA-ABC, HLA-DR, CD1α, CD40, and/or CD54. This technique can also be used to distinguish between and imDCs, using fluorochrome-labeled antibodies directed against CD14, which is present in immature, but not DCs.

### 5.3 GENERATION OF FUSION CELLS

Non-dendritic cells can be fused to autologous DCs as follows. Cells can be sterile washed prior to fusion. Fusion can be accomplished by any cell fusion technique in the art that provided that the fusion technique results in a mixture of fused cells suitable for injection into a mammal for treatment of cancer or infectious disease. Preferably, electrofusion is used. Electrofusion techniques are well known in the art (Stuhler and Walden, 1994, Cancer Immunol. Immunother. 39: 342-345; see Chang et al. (eds.), Guide to Electroporation and Electrofusion. Academic Press, San Diego, 1992).

In a preferred embodiment, the following protocol is used. In the first step, approximately 5 x 10⁷ tumor cells and 5 x 10⁷ dendritic cells (DCs) are suspended in 0.3 M glucose and transferred into an electrofusion cuvette. The sample is dielectrophoretically aligned to form cell-cell conjugates by pulsing the cell sample at 100 V/cm for 5-10 secs. Optionally, alignment may be optimized by applying a drop of dielectrical wax onto one aspect of the electroporation cuvette to 'inhomogenize' the electric field, thus directing the cells to the area of the highest field strength. In a second step, a fusion pulse is applied. Various parameters may be used for the electrofusion. For example, in one embodiment, the fusion pulse may be from a single to a triple pulse. In another embodiment, electrofusion is accomplished using from 500 to 1500V/cm, preferably,1,200V/cm at about 25 µF.

In an alternative embodiment, the following protocol is used. First, bone marrow is isolated and red cells lysed with ammonium chloride (Sigma, St. Louis, MO). Lymphocytes, granulocytes and DCs are depleted from the bone marrow cells and the remaining cells are plated in 24-well culture plates (1 x 10⁶ cells/well) in RPMI 1640 medium supplemented with 5% heat-inactivated FBS, 50 µM 2-mercaptoethanol, 2 mM glutamate, 100 U/ml penicillin, 100 pg/ml streptomycin, 10ng/ml recombinant murine granulocyte-macrophage colony stimulating factor (GM-CSF; Becton Dickinson, San Jose, CA) and 30 U/ml recombinant mouse interleukin-4 (IL-4; Becton Dickinson). Second, on day 5 of culture, nonadherent and loosely adherent cells are collected and replated on 100-mm petri dishes (1 x 10⁶ cells/mi; 10ml/dish). Next, GM-CSF and IL-4 in RPMI medium are added to the cells and 1 x 10⁶ DCs are mixed with 3 x 10⁶ irradiated (50 Gy, Hitachi MBR-1520R, dose rate: 1.1 Gy/min.) SR-B10.A cells. After 48 h, fusion is started by adding dropwise for 60 sec, 500 µl of a 50% solution of polyethylene glycol (PEG; Sigma) The fusion is stopped by stepwise addition of serum-free RPMI medium. FCs are plated in 100-mm petri dishes in the presence of GM-CSF and IL-4 in RPMI medium for 48 h.

We also describe that, the dendritic cell and the non-dendritic cell are fused as described above and subsequently, the fused cells are transfected with genetic material which encodes a molecule which stimulates a CTL and/or humoral immune response. The genetic material may be mRNA which encodes IL-12. Preferred methods of transfection include electroporation or cationic polymers.

The extent of fusion cell formation within a population of antigenic and dendritic cells can be determined by a number of diagnostic techniques known in the art. In one embodiment, for example, hybrids are characterized by emission of both colors after labeling of DCs and tumor cells with red and green intracellular fluorescent dyes, respectively. Samples of DCs without tumor cells, and tumor cells without DCs can be used as negative controls, as well as tumor + DC mixture without electrofusion.

Before introduction of the fusion cell-cytokine composition into a patient, the fusion cells are inactivated so as to prevent the tumor cells from proliferating, for example, by irradiation. Preferably, cells are irradiated at 200 Gγ, and injected without further selection. In one embodiment, the fusion cells prepared by this method comprise approximately 10 and 20% of the total cell population. In yet another embodiment, the fusion cells prepared by this method comprise approximately 5 to 50% of the total cell population.

### 5.3.1 RECOMBINANT CELLS

It is further disclosed that, rather than fusing a dendritic cell to a cancer cell or infected cell, the non-dendritic cells are transfected with a gene encoding a known antigen of a cancer or infectious agent. For example, autologous or allogeneic non-dendritic cells are isolated and transfected with a vector encoding a gene, such as for example a major antigen expressed on hepatitis B or hepatitis C. The non-dendritic cells are then selected for those expressing the recombinant antigen and administered to the patient in need thereof in combination with a cytokine or molecule which stimulates or induces a CTL and/or humoral immune response.

Recombinant expression of a gene by gene transfer, or gene therapy, refers to the administration of a nucleic acid to a subject. The nucleic acid, either directly or indirectly via its encoded protein, mediates a therapeutic effect in the subject. The present invention provides methods of gene therapy wherein genetic material, e.g., DNA or mRNA, encoding a protein of therapeutic value (preferably to humans) is introduced into the fused cells according to the methods of the invention, such that the nucleic acid is expressible by the fused cells, followed by administration of the recombinant fused cells to a subject.

The recombinant fused cells as herein discribed can be used in any of the methods for gene therapy available in the art. Thus, the nucleic acid introduced into the cells may encode any desired protein, *e*.*g*., an antigenic protein or portion thereof or a protein that stimulates a CTL and/or humoral immune response. The descriptions below are meant to be illustrative of such methods.

For general reviews of the methods of gene therapy, see Lundstrom, 1999, J. Recept. Signal Transduct. Res. 19:673-686; Robbins and Ghivizzani, 1998, Pharmacol. Ther.80:35-47; Pelegrin et al., 1998, Hum. Gene Ther. 9:2165-2175; Harvey and Caskey, 1998, Curr. Opin. Chem. Biol. 2:512-518; Guntaka and Swamynathan, 1998, Indian J. Exp. Biol. 36:539-535; Desnick and Schuchman, 1998, Acta Paediatr. Jpn. 40:191-203; Vos, 1998, Curr. Opin. Genet. Dev. 8:351-359; Tarahovsky and Ivanitsky, 1998, Biochemistry (Mosc) 63:607-618; Morishita et al., 1998, Circ. Res. 2:1023-1028; Vile et al., 1998, Mol. Med. Today 4:84-92; Branch and Klotman, 1998, Exp. Nephrol. 6:78-83; Ascenzioni et al., 1997, Cancer Lett. 118:135-142; Chan and Glazer, 1997, J. Mol. Med. 75:267-282. Methods commonly known in the art of recombinant DNA technology which can be used are described in Ausubel et al. (eds.), 1993, Current Protocols in Molecular Biology, John Wiley & Sons, NY; and Kriegler, 1990, Gene Transfer and Expression, A Laboratory Manual, Stockton Press, NY.

If recombinant cells are used in gene therapy, a gene whose expression is desired in a patient is introduced into the fused cells such that it is expressible by the cells and the recombinant cells are then administered *in vivo* for therapeutic effect.

Recombinant fused cells can be used in any appropriate method of gene therapy, as would be recognized by those in the art upon considering this disclosure. The resulting action of recombinant manipulated cells administered to a patient can, for example, lead to the activation or inhibition of a pre-selected gene, such as activation of IL-12, in the patient, thus leading to improvement of the diseased condition afflicting the patient.

The desired gene is transferred, via transfection, into fused by such methods as electroporation, lipofection, calcium phosphate mediated transfection, or viral infection. Usually, the method of transfer includes the transfer of a vector containing a selectable marker. The cells are then placed under selection to isolate those cells that have taken up and are expressing the vector, containing the selectable marker and also the transferred gene. Those cells are then delivered to a patient.

The desired gene is introduced into fused, cells prior to administration *in vivo* of the resulting recombinant cell. Such introduction can be carried out by any method known in the art, including but not limited to transfection, electroporation, microinjection, infection with a viral or bacteriophage vector containing the gene sequences, cell fusion, chromosome-mediated gene transfer, microcell-mediated gene transfer, spheroplast fusion, etc. Numerous techniques are known in the art for the introduction of foreign genes into cells (see *e*.*g*., Loeffler and Behr, 1993, Meth. Enzymol. 217:599-618; Cohen et al., 1993, Meth. Enzymol. 217:618-644; Cline, 1985, Pharmac. Ther. 29:69-92), provided that the necessary developmental and physiological functions of the recipient cells are not disrupted. The technique should provide for the stable transfer of the gene to the cell, so that the gene is expressible by the cell and preferably heritable and expressible by its cell progeny.

One common method of practicing gene therapy is by making use of retroviral vectors (see Miller et al., 1993, Meth. Enzymol. 217:581-599). A retroviral vector is a retrovirus that has been modified to incorporate a preselected gene in order to effect the expression of that gene. It has been found that many of the naturally occurring DNA sequences of retroviruses are dispensable in retroviral vectors. Only a small subset of the naturally occurring DNA sequences of retroviruses is necessary. In general, a retroviral vector must contain all of the *cis*-acting sequences necessary for the packaging and integration of the viral genome. These *cis*-acting sequences are:
a) a long terminal repeat (LTR), or portions thereof, at each end of the vector;
b) primer binding sites for negative and positive strand DNA synthesis; and
c) a packaging signal, necessary for the incorporation of genomic RNA into virions.

The gene to be used in gene therapy is cloned into the vector, which facilitates delivery of the gene into an cell by infection or delivery of the vector into the cell.

More detail about retroviral vectors can be found in Boesen et al., 1994, Biotherapy 6:291-302, which describes the use of a retroviral vector to deliver the mdrl gene to hematopoietic stem cells in order to make the stem cells more resistant to chemotherapy. Other references illustrating the use of retroviral vectors in gene therapy are: Clowes et al., 1994, J. Clin. Invest. 93:644-651; Kiem et al., 1994, Blood 83:1467-1473; Salmons and Gunzberg, 1993, Human Gene Therapy 4:129-141; and Grossman and Wilson, 1993, Curr. Opin. in Genetics and Devel. 3:110-114.

Adenoviruses can be used to deliver genes to non-dendritic cells derived from the liver, the central nervous system, endothelium, and muscle. Adenoviruses have the advantage of being capable of infecting non-dividing cells. Kozarsky and Wilson, 1993, Current Opinion in Genetics and Development 3:499-503 present a review of adenovirus-based gene therapy. Other instances of the use of adenoviruses in gene therapy can be found in Rosenfeld et al., 1991, Science 252:431-434; Rosenfeld et al., 1992, Cell 68:143-155; and Mastrangeli et al., 1993, J. Clin. Invest. 91:225-234.

It has been proposed that adeno-associated virus (AAV) be used in gene therapy (Walsh et al., 1993, Proc. Soc. Exp. Biol. Med. 204:289-300). It has also been proposed that alphaviruses be used in gene therapy (Lundstrom, 1999, J. Recept. Signal Transduct. Res. 19:673-686).

Other methods of gene delivery in gene therapy include mammalian artificial chromosomes (Vos, 1998, Curr. Op. Genet. Dev. 8:351-359); liposomes (Tarahovsky and Ivanitsky, 1998, Biochemistry (Mosc) 63:607-618); ribozymes (Branch and Klotman, 1998, Exp. Nephrol. 6:78-83); and triplex DNA (Chan and Glazer, 1997, J. Mol. Med. 75:267-282).

A desired gene can be introduced intracellularly and incorporated within host cell DNA for expression, by homologous recombination (Koller and Smithies, 1989, Proc. Natl. Acad. Sci. USA 86:8932-8935; Zijlstra et al., 1989, Nature 342:435-438).

The desired gene recombinantly expressed in the cell to be introduced for purposes of gene therapy comprises an inducible promoter operably linked to the coding region, such that expression of the recombinant gene is controllable by controlling the presence or absence the appropriate inducer of transcription.

The desired gene recombinantly expressed in the cells, whether its function is to elicit a cell fate change according to the methods of the invention, may be flanked by Cre sites. When the gene function is no longer required, the cells comprising the recombinant gene are subjected to Lox protein, for example be means of supplying a nucleic acid containing the Lox coding sequences functionally coupled to an inducible or tissue specific promoter, or by supplying Lox protein functionally coupled to a nuclear internalization signal. Lox recombinase functions to recombine the Cre sequences (Hamilton et al., 1984, J. Mol. Biol. 178:481-486), excising the intervening sequences in the process, which according to this embodiment contain a nucleic acid of a desired gene. The method has been used successfully to manipulate recombinant gene expression (Fukushige et al., 1992, Proc. Natl. Acad. Sci. USA 89:7905-7909). Alternatively, the FLP/FRT recombination system can be used to control the presence and expression of genes through site-specific recombination (Brand and Perrimon, 1993, Development 118:401-415).

Gene therapy using nucleic acids encoding hepatitis B or hepatitis C major antigens are directed to the treatment of viral hepatitis is also described.

### 5.4 IMMUNE CELL ACTIVATING MOLECULES

A composition is described which comprises first, a fusion cell derived from the fusion of a dendritic and non-dendritic cell, and second, a cytokine or other molecule which can stimulate or induce a cytotoxic T cell (CTL) response.

IL-12 plays a major role in regulating the migration and proper selection of effector cells in an immune response. The IL-12 gene product polarizes the immune response toward the TH₁ subset of T helper cells and strongly stimulates CTL activity. In a preferred embodiment, the CTL stimulating molecule is IL-12. As elevated doses of IL-12 exhibits toxicity when administered systemically, IL-12 is preferably administered locally. Additional modes of administration are described below in Section 5.7.1.

Expression of IL-12 receptor β2 (IL-12R-β2) is necessary for maintaining IL-12 responsiveness and controlling TH₁ lineage commitment. Furthermore, IL-12 signaling results in STAT4 activation, *i*.*e*., measured by an increase of phosphorylation of STAT4, and interferon-γ (IFN-γ) production. Thus, the use of a molecule, which is not IL-12, which can activate STAT4, for example a small molecule activator of STAT4 identified by the use of combinatorial chemistry is described.

The immune stimulating molecule may be IL-18 or IL-15 or interferon-γ.

The subject to be treated is given any combination of molecules or cytokine described herein which stimulate or induce a CTL and/or humoral immune response.

To increase the cytotoxic T-cell pool, *i*.*e*., the TH₁ cell subpopulation, anti-IL-4 antibodies can be added to inhibit the polarization of T-helper cells into TH₂ cells, thereby creating selective pressure toward the TH₁ subset of T-helper cells. Further, anti-IL-4 antibodies can be administered concurrent with the administration of IL-12, to induce the TH cells to differentiate into TH₁ cells. After differentiation, cells can be washed, resuspended in, for example, buffered saline, and reintroduced into a patient via, preferably, intravenous administration.

In the context of the present description, also variants of the above-described interleukins are described. Such variants have an altered amino acid sequence which can function as agonists (mimetics) to promote a CTL and/or humoral immune response response. Variants can be generated by mutagenesis, *e*.*g*., discrete point mutation or truncation. An agonist can retain substantially the same, or a subset, of the biological activities of the naturally occurring form of the protein. An antagonist of a protein can inhibit one or more of the activities of the naturally occurring form of the protein by, for example, competitively binding to a downstream or upstream member of a cellular signaling cascade which includes the protein of interest. Thus, specific biological effects can be elicited by treatment with a variant of limited function. Treatment of a subject with a variant having a subset of the biological activities of the naturally occurring form of the protein can have fewer side effects in a subject relative to treatment with the naturally occurring form of the protein.

Variants of a molecule capable of stimulating a CTL and/or humoral immune response can be identified by screening combinatorial libraries of mutants, *e*.*g*., truncation mutants, for agonist activity. In one embodiment, a variegated library of variants is generated by combinatorial mutagenesis at the nucleic acid level and is encoded by a variegated gene library. A variegated library of variants can be produced by, for example, enzymatically ligating a mixture of synthetic oligonucleotides into gene sequences such that a degenerate set of potential protein sequences is expressible as individual polypeptides, or alternatively, as a set of larger fusion proteins (*e*.*g*., for phage display). There are a variety of methods which can be used to produce libraries of potential variants of IL-12 from a degenerate oligonucleotide sequence. Methods for synthesizing degenerate oligonucleotides are known in the art (*see, e*.*g*., Narang, 1983, Tetrahedron 39:3; Itakura et al., 1984, Annu. Rev. Biochem., 53:323; Itakura et al., 1984, Science, 198:1056; Ike et al., 1983, Nucleic Acid Res., 11:477).

In addition, libraries of fragments of the coding sequence of an interleukin capable of promoting a CTL and/or humoral immune response can be used to generate a variegated population of polypeptides for screening and subsequent selection of variants. For example, a library of coding sequence fragments can be generated by treating a double stranded PCR fragment of the coding sequence of interest with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S 1 nuclease, and ligating the resulting fragment library into an expression vector. By this method, an expression library can be derived which encodes N-terminal and internal fragments of various sizes of the protein of interest.

Several techniques are known in the art for screening gene products of combinatorial libraries made by point mutations or truncation, and for screening cDNA libraries for gene products having a selected property. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a technique which enhances the frequency of functional mutants in the libraries, can be used in combination with the screening assays to identify variants of an interleukin capable of promoting a CTL and/or humoral immune response (Arkin and Yourvan, 1992, Proc. Natl. Acad. Sci. USA, 89:7811-7815; Delgrave et al.,1993, Protein Engineering, 6(3):327-331).

### 5.5 ASSAYS FOR MEASURING AN IMMUNE RESPONSE

The fusion cell-cytokine compositions can be assayed for immunogenicity using any method known in the art. By way of example but not limitation, one of the following procedures can be used.

A humoral immune response can be measured using standard detection assays including but not limited to an ELISA, to determine the relative amount of antibodies which recognize the target antigen in the sera of a treated subject, relative to the amount of antibodies in untreated subjects. A CTL response can be measured using standard immunoassays including chromium release assays as described herein. More particularly, a CTL response is determined by the measurable difference in CTL activity upon administration a stimulator, relative to CTL activity in the absence of a stimulator.

### 5.5.1 MLTC ASSAY

The fusion cell-cytokine compositions may be tested for immunogenicity using a MLTC assay. For example, 1x10⁷ fusion cells are γ-irradiated, and mixed with T lymphocytes. At various intervals the T lymphocytes are tested for cytotoxicity in a 4 hour ⁵¹Cr-release assay (see Palladino et al., 1987, Cancer Res. 47:5074-5079). In this assay, the mixed lymphocyte culture is added to a target cell suspension to give different effector:target (E:T) ratios (usually 1:1 to 40:1). The target cells are prelabelled by incubating 1x10⁶ target cells in culture medium containing 500 µCi ⁵¹Cr/ml for one hour at 37°C. The cells are washed three times following labeling. Each assay point (E:T ratio) is performed in triplicate and the appropriate controls incorporated to measure spontaneous ⁵¹Cr release (no lymphocytes added to assay) and 100% release (cells lysed with detergent). After incubating the cell mixtures for 4 hours, the cells are pelletted by centrifugation at 200g for 5 minutes. The amount of ⁵¹Cr released into the supernatant is measured by a gamma counter. The percent cytotoxicity is measured as cpm in the test sample minus spontaneously released cpm divided by the total detergent released cpm minus spontaneously released cpm.

In order to block the MHC class I cascade a concentrated hybridoma supernatant derived from K-44 hybridoma cells (an anti-MHC class I hybridoma) is added to the test samples to a final concentration of 12.5%.

### 5.5.2 ANTIBODY RESPONSE ASSAY

The immunogenicity of fusion cells may be determined by measuring antibodies produced in response to the vaccination, by an antibody response assay, such as an enzyme-linked immunosorbent assay (ELISA) assay. Methods for such assays are well known in the art (see, e.g., Section 2.1 of Current Protocols in Immunology, Coligan et al. (eds.), John Wiley and Sons, Inc. 1997). Microtitre plates (96-well Immuno Plate II, Nunc) are coated with 50 µl/well of a 0.75 µg/ml solution of a purified cancer cell or infected used in the composition in PBS at 4°C for 16 hours and at 20°C for 1 hour. The wells are emptied and blocked with 200 µl PBS-T-BSA (PBS containing 0.05% (v/v) TWEEN 20 and 1% (w/v) bovine serum albumin) per well at 20°C for 1 hour, then washed 3 times with PBS-T. Fifty µl/well of plasma or CSF from a vaccinated animal (such as a model mouse or a human patient) is applied at 20°C for 1 hour, and the plates are washed 3 times with PBS-T. The antigen antibody activity is then measured calorimetrically after incubating at 20°C for 1 hour with 50µl/well of sheep anti-mouse or anti-human immunoglobulin, as appropriate, conjugated with horseradish peroxidase diluted 1:1,500 in PBS-T-BSA and (after 3 further PBS-T washes as above) with 50 µl of an o-phenylene diamine (OPD)-H₂O₂ substrate solution. The reaction is stopped with 150 µl of 2M H₂SO₄ after 5 minutes and absorbance is determined in a photometer at 492 nm (ref. 620 nm), using standard techniques.

### 5.5.3 CYTOKINE DETECTION ASSAYS

The CD4⁺ T cell proliferative response to the fusion cell-cytokine composition may be measured by detection and quantitation of the levels of specific cytokines. In one embodiment, for example, intracellular cytokines may be measured using an IFN-γ detection assay to test for immunogenicity of the fusion cell-cytokine composition. In an example of this method, peripheral blood mononuclear cells from a patient treated with the fusion cell-cytokine composition are stimulated with peptide antigens such as mucin peptide antigens or Her2/neu derived epitopes. Cells are then stained with T cell-specific labeled antibodies detectable by flow cytometry, for example FITC-conjugated anti-CD8 and PerCP-labeled anti-CD4 antibodies. After washing, cells are fixed, permeabilized, and reacted with dye-labeled antibodies reactive with human IFN-γ (PE- anti-IFN-y). Samples are analyzed by flow cytometry using standard techniques.

Alternatively, a filter immunoassay, the enzyme-linked immunospot assay (ELISPOT) assay, may be used to detect specifc cytokines surrounding a T cell. In one embodiment, for example, a nitrocellulose-backed microtiter plate is coated with a purified cytokine-specific primary antibody, *i*.*e*., anti-IFN-γ, and the plate is blocked to avoid background due to nonspecific binding of other proteins. A sample of mononuclear blood cells, containing cytokine-secreting cells, obtained from a patient vaccinated with a fusion cell-cytokine composition, is diluted onto the wells of the microtitre plate. A labeled, e.g., biotin-labeled, secondary anti-cytokine antibody is added. The antibody cytokine complex can then be detected, *i*.*e*. by enzyme-conjugated streptavidin - cytokine-secreting cells will appear as "spots" by visual, microscopic, or electronic detection methods.

### 5.5.4 TETRAMER STAINING ASSAY

The "tetramer staining" assay (Altman et al., 1996, Science 274: 94-96) may be used to identify antigen-specific T-cells. For example, an MHC molecule containing a specific peptide antigen, such as a tumor-specific antigen, is multimerized to make soluble peptide tetramers and labeled, for example, by complexing to streptavidin. The MHC complex is then mixed with a population of T cells obtained from a patient treated with a fusion cell composition. Biotin is then used to stain T cells which express the antigen of interest, *i*.*e*., the tumor-specific antigen.

Cytotoxic T-cells are immune cells which are CD8 positive and have been activated by antigen presenting cells (APCs), which have processed and are displaying an antigen of a target cell. The antigen presentation, in conjunction with activation of co-stimulatory molecules such as B-7/CTLA-4 and CD40 leads to priming of the T-cell to target and destroy cells expressing the antigen.

Cytotoxic T-cells are generally characterized as expressing CD8 in addition to secreting TNF-β, perforin and IL-2. A cytotoxic T cell response can be measured in various assays, including but not limited to increased target cell lysis in ⁵¹Cr release assays using T-cells from treated subjects, in comparison to T-cells from untreated subjects, as shown in the examples herein, as well as measuring an increase in the levels of IFN-γ and IL-2 in treated subjects relative to untreated subjects.

### 5.6 TARGET CANCERS

The cancers and oncogenic diseases that can be treated or prevented using the fusion cells of the invention of the present invention include, but are not limited to: human sarcomas and carcinomas, *e*.*g*., , renal cell carcinoma, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma; leukemias, *e*.*g*., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenström's macroglobulinemia, and heavy chain disease.

### 5.7 TARGET INFECTIOUS DISEASES

The infectious diseases that can be treated or prevented using the fusion cells as herein described include those caused by intracellular pathogens such as viruses, bacteria, protozoans, and intracellular parasites. Viruses include, but are not limited to viral diseases such as those caused by hepatitis type B virus, parvoviruses, such as adeno-associated virus and cytomegalovirus, papovaviruses such as papilloma virus, polyoma viruses, and SV40, adenoviruses, herpes viruses such as herpes simplex type I (HSV-I), herpes simplex type II (HSV-II), and Epstein-Barr virus, poxviruses, such as variola (smallpox) and vaccinia virus, RNA viruses, including but not limited to human immunodeficiency virus type I (HIV-I), human immunodeficiency virus type II (HIV-II), human T-cell lymphotropic virus type I (HTLV-I), and human T-cell lymphotropic virus type II (HTLV-II); influenza virus, measles virus, rabies virus, Sendai virus, picomaviruses such as poliomyelitis virus, coxsackieviruses, rhinoviruses, reoviruses, togaviruses such as rubella virus (German measles) and Semliki forest virus, arboviruses, and hepatitis type A virus.

Bacterial infections can be treated or prevented such as, but not limited to disorders caused by pathogenic bacteria including, but not limited to, *Streptococcus pyogenes, Streptococcus pneumoniae, Neisseria gonorrhoea, Neisseria meningitidis, Corynebacterium diphtheriae, Clostridium botulinum, Clostridium perfringens, Clostridium tetani, Haemophilus influenzae, Klebsiella pneumoniae, Klebsiella ozaenae, Klebsiella rhinoscleromotis, Staphylococcus aureus, Vibrio cholerae, Escherichia coli, Pseudomonas aeruginosa, Campylobacter (Vibrio) fetus, Campylobacter jejuni, Aeromonas hydrophila, Bacillus cereus, Edwardsiella tarda, Yersinia enterocolitica, Yersinia pestis, Yersinia pseudotuberculosis, Shigella dysenteriae, Shigella flexneri, Shigella sonnei, Salmonella typhimurium, Salmonella typhii, Treponema pallidum, Treponema pertenue, Treponema carateneum, Borrelia vincentii, Borrelia burgdorferi, Leptospira icterohemorrhagiae, Mycobacterium tuberculosis, Toxoplasma gondii, Pneumocystis carinii, Francisella tularensis, Brucella abortus, Brucella suis, Brucella melitensis, Mycoplasma spp., Rickettsia prowazeki, Rickettsia tsutsugumushi, Chlamydia spp.,* and *Helicobacter pylori.*

The methods can be used to treat or prevent infections caused by pathogenic protozoans such as, but not limited to, *Entomoeba histolytica, Trichomonas tenas, Trichomonas hominis, Trichomonas vaginalis, Trypanosoma gambiense, Trypanosoma rhodesiense, Trypanosoma cruzi, Leishmania donovani, Leishmania tropica, Leishmania braziliensis, Pneumocystis pneumonia, Plasmodium vivax, Plasmodium falciparum,* and *Plasmodium malaria*.

### 5.8 PHARMACEUTICAL PREPARATIONS AND METHODS OF ADMINISTRATION

The composition formulations of the invention are defined in the claims and comprise an effective immunizing amount of the fusion cells which are to be administered with a molecule capable of stimulating a CTL and/or humoral immune response, namely IL-12.

Suitable preparations of fusion cell-cytokine compositions include injectables, preferably as a liquid solution.

Many methods may be used to introduce the composition formulations of the invention; these include but are not limited to subcutaneous injection, intralymphatically, intradermal, intramuscular, intravenous, and via scarification (scratching through the top layers of skin, e.g., using a bifurcated needle). Preferably, fusion cell-cytokine compositions are injected intradermally.

In addition, if desired, the composition preparation may also include minor amounts of auxiliary substances such as wetting or emulsifying agents, pH buffering agents, and/or compounds which enhance the effectiveness of the composition. The effectiveness of an auxiliary substances may be determined by measuring the induction of antibodies directed against a fusion cell.

The mammal to which the composition is administered is preferably a human, but can also be a non-human animal including but not limited to cows, horses, sheep, pigs, fowl (*e.g.*, chickens), goats, cats, dogs, hamsters, mice and rats.

### 5.9 EFFECTIVE DOSE

The compositions can be administered to a patient at therapeutically effective doses to treat or prevent cancer or infectious disease. A therapeutically effective amount refers to that amount of the fusion cells sufficient to ameliorate the symptoms of such a disease or disorder, such as, e.g., regression of a tumor. Effective doses (immunizing amounts) of the compositions of the invention may also be extrapolated from dose-response curves derived from animal model test systems. The precise dose of fusion cells to be employed in the composition formulation will also depend on the particular type of disorder being treated. For example, if a tumor is being treated, the aggressiveness of the tumor is an important consideration when considering dosage. Other important considerations are the route of administration, and the nature of the patient. Thus the precise dosage should be decided according to the judgment of the practitioner and each patient's circumstances, e.g., the immune status of the patient, according to standard clinical techniques.

For example, to treat a human tumor, a fusion cell-cytokine composition formed by cells of the tumor fused to autologous DCs at a site away from the tumor, and preferably near the lymph tissue. The administration of the composition may be repeated after an appropriate interval, *e.g.*, every 3-6 months, using approximately 1 x 10⁸ cells per administration.

### 5.10 KITS

Further described are kits for facilitating delivery of the immunotherapeutic according to the methods of the invention. The kits described herein may be conveniently used, *e.g*., in clinical settings to treat patients exhibiting symptoms of cancer of an infectious disease. For example, a kit is provided comprising, in one or more containers: a) a sample of a population of dendritic cells and b) instructions for its use in a method for treating or protecting against cancer or an infectious disease. An ampoule of sterile diluent can be provided so that the ingredients may be mixed prior to administration. The kit may further comprise a cuvette suitable for electrofusion. The dendritic cells may be cryopreserved.

### 6. EXAMPLE: VACCINATION WITH DENDRITIC CELLS AND GLIOMA CELLS AGAINST BRAIN TUMORS

In the present example, the therapeutic use of dendritic cells fused to glioma cells against tumors in the brain, an immunologically privileged site, was investigated. Prior immunization with fusion cells (FCs) resulted in prevention of tumor formation upon challenge with glioma cells in the flank or in the brain. Efficacy was reduced when studies were performed in mice depleted of CD8⁺ cells. In a treatment model, FCs were injected subcutaneously after tumor development in the brain. Administration of FCs alone had limited effects on survival of brain tumor-bearing mice. Importantly, however, administration of FCs and recombinant IL-12 (rIL-12) remarkably prolonged survival of mice with brain tumors. CTL activity against glioma cells from immunized mice was also stimulated by co-administration of FCs and rIL-12 compared with that obtained with FCs or rIL-12 alone. These data support the therapeutic efficacy of combining fusion cell-based vaccine therapy and rIL-12.

### 6.1 MATERIALS AND METHODS

### Cell lines, agents and animals

The mouse glioma cell line, SR-B10.A, was maintained as monolayer cultures in DMEM (Cosmo Bio, Tokyo, Japan) supplemented with 100 U/ml penicillin, 0.1 mg/ml streptomycin, and 10% heat-inactivated fetal bovine serum (FBS; GIBCO, Gaithersburg, MD). Yac-1 cells, obtained from RIKEN CELL BANK (Tsukuba, Japan), were maintained in RPMII640 (Cosmo Bio) with 10% FBS.

Recombinant mouse IL-12 (rmIL-12) was kindly provided by Genetics Institute, Cambridge, MA.

Female B10.A mice, purchased from Sankyo Laboratory Inc. (Shizuoka, Japan), were maintained in a specific pathogen-free room at 25±3°C. Mice were used at 8 weeks of age.

### Fusions of dendritic and tumor cells

Bone marrow was flushed from long bones of B10.A mice, and red cells were lysed with ammonium chloride (Sigma, St. Louis, MO). Lymphocytes, granulocytes and DCs were depleted from the bone marrow cells and the cells were plated in 24-well culture plates (1 x 10⁶ cells/well) in RPMI 1640 medium supplemented with 5% heat-inactivated FBS, 50 µM 2-mercaptoethanol, 2 mM glutamate, 100 U/ml penicillin, 100 pg/ml streptomycin, 10ng/ml recombinant murine granulocyte-macrophage colony stimulating factor (GM-CSF; Becton Dickinson, San Jose, CA) and 30 U/ml recombinant mouse interleukin-4 (IL-4; Becton Dickinson). On day 5 of culture, nonadherent and loosely adherent cells were collected and replated on 100-mm petri dishes (1 x 10⁶ cells/mi; 10 ml/dish). GM-CSF and IL-4 in RPMI medium were added to the cells and 1 x 10⁶ DCs were mixed with 3 x 10⁶ irradiated (50 Gy, Hitachi MBR-1520R, dose rate: 1.1 Gy/min.) SR-B10.A cells. After 48 h, fusion was started by adding dropwise for 60 sec, 500 µl of a 50% solution of polyethylene glycol (PEG; Sigma). The fusion was stopped by stepwise addition of serum-free RPMI medium. FCs were plated in 100-mm petri dishes in the presence of GM-CSF and IL-4 in RPMI medium for 48 h.

### Flow cytometry

Tumor cells (3 x10⁶) were harvested and washed twice with phosphate-buffered saline (PBS; Cosmo Bio). PKH26 (2 µl;Sigma) was added to the tumor cells and the mixture was kept at room temperature for 5 mm. Then, 500 µl FBS was added to stop the reaction. Cells were washed twice using PBS and resuspended in 500 µl of PBS. Single cell suspensions of DCs and FCs were prepared, washed, resuspended in buffer (1% BSA, 0.1% Sodium azide in PBS) and stained with an FITC-labeled anti-mouse CD80 monoclonal antibody (Pharmingen, San Diego, CA) for 30 mm at 4°C. Stained cells were analyzed using a FACScan flow cytometer (Becton Dickinson, San Jose, CA).

### Animal models

FCs were washed twice with PBS, then suspended in PBS at a density of 1 x 10⁶ ml. FCs (3 x10⁵) were subcutaneously (s.c.) inoculated into the flank of B10.A mice on days 0 and 7. Subsequently, tumor cells (1x10⁶) were inoculated s.c. into the opposite flank on day 14. In the brain tumor model, 1 x 10⁴ SR-B10. A tumor cells were stereotactically inoculated into the right frontal lobes of the brains of syngeneic mice on day 14 after immunization with FCs.

In the treatment model, 1 x 10⁴ tumor cells were stereotactically inoculated into the brains (day 0) followed by s.c. injection of FCs (3 x10⁵) on days 5 and 12. In certain experiments, rmIL-12 was injected intraperitoneally (i.p.). Autopsy was performed on deceased mice.

### Assay of cytolytic activity

The cytolytic activity of activated spleen cells (SPC) was tested *in vitro* in a ⁵¹Cr release assay. Single cell suspensions of SPC from individual mice were washed and resuspended in 10% FCS-RPMI at a density of 1 x 10⁷/ml in six-well plates (Falcon Labware, Lincoln Park, NJ) (Day 0). After removing adherent cells, 10 U/ml of recombinant human IL-2 was added to the cultures every other day. Four days after culture initiation, cells were harvested and cytotoxic T cells (CTL) activity was determined. Target cells were labeled by incubation with ⁵¹Cr for 90 mm at 37°C, then co-cultured with effector lymphocytes for 4 hours. The effector:target ratio ranged from 10:1 1 to 80:1. All determinations were made in triplicate and percentage lysis was calculated using the formula: (experimental cpm - spontaneous cpm / maximum cpm - spontaneous cpm) x 100%.

### Antibody ablation studies

*In vivo* ablation ofT-cell subsets was accomplished as previously described (Kikuchi et al., 1999, Int J Cancer, 80:425-430). Briefly, 3 x10⁵FCs were inoculated subcutaneously into the flank of B10.A mice on days 0 and 7. Subsequently, tumor cells (1 x10⁶) were inoculated into the opposite flank on day 14. The rat monoclonal antibodies anti-mCD4 (ATCC hybridoma GK1.5), anti-mCD8 (ATCC hybridoma 56.6.73), anti-asialo GMI (Wako Pure Chemicals, Tokyo, Japan) or normal rat lgG was injected i.p. (0.5 mg/injection/mouse) on days 7, 10, 14 and 17.

### Immunofluorescence staining

Tumor cells (1 x 10⁴) were stereotactically inoculated into the brains (day 0) followed by subcutaneous (s.c.) injection of FCs (3 x10⁵) or irradiated glioma cells (3 x 10⁵) on day 3 as a control. After sacrificing the mouse on day 17, we fixed the brain in fixation buffer (1% paraformaldehyde and 0.1 % glutaraldehyde in PBS) for 10 mm. Sections (6 µm thickness) were incubated overnight at 4°C with the first antibody, anti-glial fibrillary acidic protein (anti-GFAP; Zymed Laboratories, San Francisco, CA). The primary antibody was detected by FITC-conjugated goat anti-rabbit lgG (Jackson ImmunoResearch Laboratories, West Grove, PA) in a 2 h incubation at room temperature. Subsequently, sections were incubated overnight at 4°C with anti-CD4-PE (Pharmingen) or anti-CD8-PE (Pharmingen) antibody.

### Data analysis

Calculated tumor sizes were compared using a two-sample t test. Survival was evaluated by generation of Kaplan-Meier cumulative hazard plots and Wilcoxon analysis. Differences were considered significant at p < 0.05.

### 6.2 RESULTS

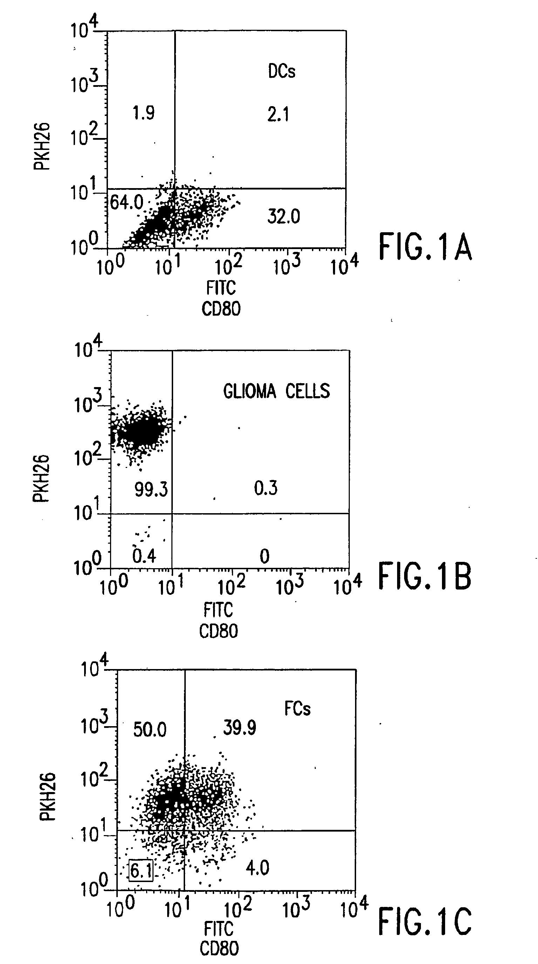

DCs and glioma cells were fused after incorporation of PKH26 into glioma cells. DCs were stained by FITC-labeled anti-CD80 monoclonal antibody. Figure 1A shows that 34% of DCs were stained by anti-CD80 monoclonal antibody. More than 95% of glioma cells were positive for PKH26 (Figure 1B). The percentage of double positive cells (39.9%; Figure 1C) was nearly identical to the percent of CD80-positive DCs and 10% of FCs were PKH26-negative, suggesting that most DCs were fused with glioma cells.

The antitumor effects of prior immunization with FCs on subcutaneous gliomas was examined. FCs, DCs, or irradiated parental cells as a control (1x10⁶) were injected s.c. into syngeneic mice on days 0 and 7 (n=11 in each group). On day 14, 1 x 10⁶ parental cells were inoculated s.c. into the opposite flank. Within two weeks, the inoculated tumor cells caused large tumors in all mice injected with irradiated parental cells. All of the mice died within six weeks. In contrast, none of the mice immunized with FCs died within six weeks. Whereas six of 11 mice immunized with DCs developed tumors, none of 11 mice immunized with FCs developed a palpable tumor (Figure 2A).

We also investigated the antitumor effects of prior immunization with FCs on gliomas in the brain. After immunization with FCs on days 0 and 7, 1 x 10⁴ tumor cells were stereotactically inoculated into the right frontal lobe of the brain (day 14). These mice were observed for 70 days. Half of the mice immunized with FCs survived longer than 70 days (n = 20 in each group; p < 0.01) (Figure 2B). All control mice died within 6 weeks. Autopsy was performed on all mice. Large tumors had developed in the dead mice, but not in the surviving mice. These findings indicate that immunization with FCs prevents the development of glioma cell tumor in the flank and in the brain.

As an experimental treatment model, FCs were injected after brain tumor development. Tumor cells (1 x 10⁴) were stereotactically inoculated into the right frontal lobes of the brains of syngeneic mice (day 0). On days 5 and 12, 3 x 10⁵ FCs were inoculated s.c.. Although. vaccination with FCs prolonged the survival of tumor-bearing mice (n = 15 each; Figure 3), the difference was not significant (p > 0.05). Inoculation of DCs alone had no effect on survival (data not shown). We then analyzed antitumor effects of combined FCs and rmIL-12 therapy. Tumor cells (1 x 10⁴) were stereotactically inoculated into the brains of syngeneic mice (day 0). On days 5 and 12, 3 x 10⁵ FCs were inoculated s.c.. All mice were given an i.p. injection of 0.5 µg/100 µl rmIL-12 or 100 µl saline every other day for two weeks (3.5 µg/mouse total) starting on day 5. Vaccination with both FCs and rIL-1 2 prolonged survival in comparison with the control (p = 0.01; Figure 3). Five of ten mice treated with FCs and rIL-12 survived over 70 days. The difference in survival rates between the controls and mice treated with rmIL-12 alone or both DCs and rmIL-12 was not statistically significant (data not shown). These results demonstrate that rmIL-12 potentiates the antitumor effects of the FC composition.

CTL activity was analyzed by a ⁵¹Cr release assay. After immunization with FCs (on day 0 and/or 7) and/or rIL-12 (every other day for 10 days starting on day 7; 2.5 pg/mouse total), splenocytes (SPCs) were separated from untreated mice and the mice immunized with FCs once or twice. Figure 4 shows that CTL activity on tumor cells from immunized mice, especially mice injected with rIL-12 and immunized with FCs twice, was considerably increased compared with the control and others and that antitumor activity on Yac-1 cells from treated mice did not significantly increase (data not shown). These results suggest that vaccination with FCs induced antitumor activity and that the cytolytic activity of SPCs from treated mice was tumor-specific.

In addition, lymphocyte subsets were depleted by administering anti-CD4, anti-CD8, anti-asialo GMI, or control rat lgG into mice given injections of glioma cells and FCs. On days 0 and 7, FCs were subcutaneously inoculated into the flank. Subsequently, on day 14 parental cells were inoculated into the opposite flank. The mAbs were injected i.p. on days 7, 10, 14, and 17. The antitumor effect was reduced in mice depleted of CD8⁺ T cells (n = 4 in each group; Figure 5). The protection conferred by FCs was not abolished by CD4⁺T or NK cell depletion. These results demonstrate that CD8⁺ T cells are required for the antitumor effect of FCs in this model.

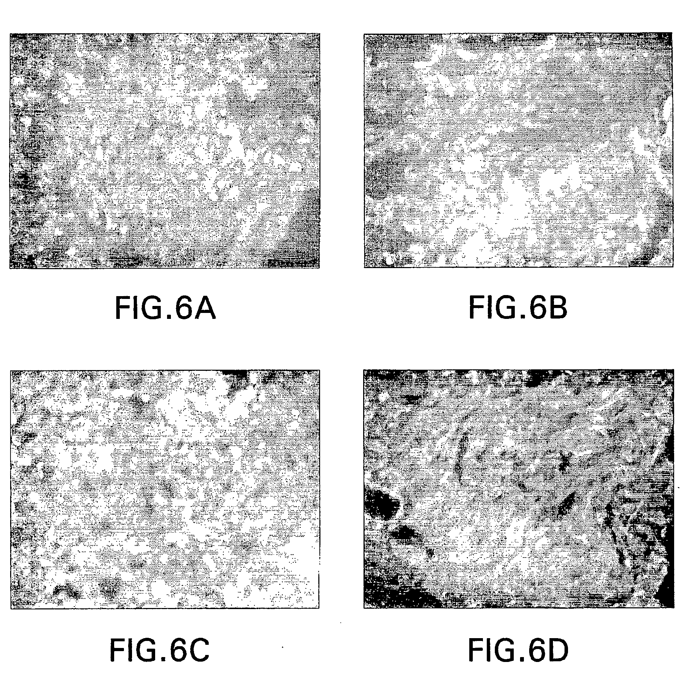

In the experimental treatment model, we analyzed whether CD4⁺ and/or CD8⁺ T cells were infiltrating into the brain tumor. Immunofluorescence analysis of the brain tumors showed that a few CD4⁺ and CD8⁺ T cells were present in the tumors of non-vaccinated mice (Figure 6A, B). In contrast, numerous CD4⁺ and CD8⁺ T cells were detectable in the tumors of vaccinated mice (Figure 6C, D). As reported previously; SR-B 10.A cells were positive for GFAP (10).

### 6.3 DISCUSSION

Genetically engineered glioma cells can be used as APCs for vaccination against gliomas, but the antitumor effect is not sufficient to eradicate established brain tumors in the mouse model (Aoki et al., 1992, Proc Natl Acad Sci U S A, 89:3850-4); Wakimoto, H. et al., 1996, Cancer Res, 56:1828-33). Therefore, a DC-based composition is a potential approach that could be used for the treatment of brain tumors. DCs lose the ability to take up antigens. Therefore, use of DCs requires efficient methods to incorporate TAAs into DCs. So far, several methods using DCs for the induction of antitumor immunity have been investigated: DCs pulsed with proteins or peptides extracted from tumor cells (Zitvogel et al., 1996; Nair et al., 1997, Int J Cancer, 70:706-15; Tjandrawan et al., 1998, J Immunother, 21:149-57), QCs transfected with genes encoding TAAs (Tuting et al., 1998, J Immunol, 160:1139-47), DCs cultured with tumor cells (Celluzi and Falo, 1998) and DCs fused with tumor cells (Gong et al.,1997, Nat Med, 3:558-61; Gong et al., 1998, Proc Natl Acad Sci U S A, 95:6279-83; Lespagnard et al., 1998, Int J Cancer, 76:250-8; Wang et al., 1998, J Immunol, 161:5516-24). Since, 1) FCs can be used to induce antitumor immunity against unknown TAAs, 2) the common TAAs of gliomas have not been identified and 3) antitumor effects of FCs provide a more thorough cure than mixture of DCs and tumor cells, FCs may have an advantage as a potential therapeutic approach for malignant gliomas.

Although the effects of FCs on tumor cells in a mouse subcutaneous tumor model were previously reported (Gong et al., 1997, Nat Med, 3:558-61), the effects in the brain remained unclear. In our brain tumor model, systemic vaccination with FCs rendered tumor cells susceptible to rejection, which resulted in the establishment of systemic immunity and prolonged survival. The central nervous system (CNS) is generally considered an immunologically privileged site due to the lack of lymphatic drainage and the nature of the blood brain barrier in which tight junctions between cerebral vascular endothelial cells form a physical barrier to the passage of cells and antibodies (Cserr, H.F. and Knopf, P.M., 1992, Immunol Today, 13:507-12). However, the present study shows that systemic vaccination with FCs can be used to treat established brain tumors. Therefore, the brain may not be completely immuno-privileged or, alternatively, barriers to the immune system can be surmounted for certain tumors, resulting in crosstalk between systemic and focal immunity.

In the present study, vaccination with FCs alone prolonged survival of mice with brain tumors. We therefore reasoned that the immunization treatment schedule and method might be improved by injecting FCs with stimulatory cytokines. Indeed, administration of rmIL-12 enhanced the antitumor effect of FCs against mouse gliomas. IL-12, originally called natural killer cell stimulatory factor or cytotoxic lymphocyte maturation factor, enhances the lytic activity of NK/lymphokine-activated killer (LAK) cells, facilitates specific cytotoxic T lymphocyte (CTL) responses, acts as a growth factor for activated T and NK cells, induces production of IFN-γ from T and NK cells, and acts as an angiogenesis inhibitor (Brunda, M.J., 1994, J. Leukoc Biol, 55:280-8). Although IL-12 has the potential to be used as an immunomodulator in the therapy of malignancies and has been shown to significantly retard the growth of certain murine tumors (Gately et al., 1994, Int Immunol, 6:157-67); Nastala et al., 1994, J Immunol, 153:1697-706), systemic administration of rmIL-12 did not prolong the survival of mice with brain tumors (Kikuchi et al., 1999, Int J Cancer, 80:425-430), indicating that the antitumor effect of combined FCs and rmIL-12 therapy may be synergistic. There were few lymphocytes present in the brain tumors from control mice. Importantly, however, immunized with FCs substantially increased lymphocyte infiltration. In addition, at the tumor site, the concentration of tumor-derived immuno-suppressive factors (e.g. TGF-β, IL-10, prostaglandin E2) may be high, indicating that more potent CTL may be needed to cure brain tumors.

DCs can sensitize CD4⁺ T cells to specific antigens in a MHC-restricted manner. CD4⁺ T cells are critical in priming both cytotoxic T lymphocytes and antigen non-specific effector immune responses, implying that both CD4⁺ and CD8⁺ T cells are equally important in antitumor immunity. As reported previously, antitumor effects of cells fused with DCs and MC38 were mediated via both CD4⁺ and CD8⁺ T cells (Gong et al., 1997, Nat Med, 3:558-61). However, our results demonstrated that CD8⁺ T cells were required for the antitumor effect of FCs and that the role of CD4⁺ T cells less obvious. Okada et al. (1998, Int J Cancer, 78:196-201) reported.that only CD8⁺ T cells were required for antitumor effects of peptide-pulsed DCs in a brain tumor model (Okada et al., 1998, Int J Cancer, 78: 196-201). Therefore, the cell type mediating the anti-tumor effects of DCs may not be universal, but rather dependent upon the experimental model. Histopathological findings showed that both CD4⁺ and CD8⁺ T cells were present in the brain tumors. It may be speculated that CTLs were already primed before starting the vaccination with FCs. That is, CD4⁺ T cells have already finished priming CTLs before immunization with FCs and pre-CTLs (primed CTLs) were stimulated by FCs, resulting in induction of activated CTLs and acquisition of antitumor activity.

In conclusion, our data suggest that vaccination with FCs and rIL-12 can be used to treat malignant gliomas in a mouse model. In the present study, we fused DCs with an established tumor cell line. However, for clinical application, DCs should be fused with removed tumor materials or primary cultured cells. Future research will focus on characterizing the antitumor activities of cells fused with DCs and primary cultured human glioma cells.

### 7. EXAMPLE: TREATMENT WITH TUMOR CELL-DENDRITIC CELL HYBRIDS IN COMBINATION WITH INTERLEUKIN-12

Hepatocellular carcinoma (HCC) is one of the most common cancers in the world, especially in Asian and African countries. While this disease is rare elsewhere (a), recent reports have indicated that HCC is now increasing in Western countries (El-Selag et al., 1999, N. Engl. J. Med., 340:745-750). Epidemiological and prospective studies have demonstrated a strong etiological association between hepatitis B virus (HBV) and/or hepatitis C virus (HCV) infection and HCC (Ikeda et al., 1993, Hepatology, 18:47-5; Obata et al., 1980, Int. J. Cancer, 25:741-747; Saito et al., 1990, Proc. Natl. Acad. Sci. USA, 87:6547-6549). In Japan, about 76% of HCC patients had chronic HCV infection and 78% of them had liver cirrhosis (Liver Cancer Study Group of Japan, 1998). The reduction in functional reserve due to the coexisting liver cirrhosis has limited surgical resection of the tumor. Consequently, treatment has involved cancer chemotherapy, transcatheter arterial embolization, transcatheter arterial chemotherapy, percutaneous ethanol injection and percutaneous microwave coagulation therapy. However, the recurrence rate after these therapies is high (Liver Cancer Study Group of Japan, 1998; Tarao et al., Cancer, 79:688-694), probably because of the insufficient therapeutic effect and multicentric development of HCC in a cirrhotic liver.

In the present study, we show that the growth of HCC tumors is prevented by vaccination of DCs fused to HCC cells prior to inoculation of HCC cells. In addition, treatment of established HCC tumors with DC/HCC requires co-administration with IL-12. Importantly, IL- 12 can also enhance the effectiveness of fusion cell-based immunotherapy.

### 7.1 MATERIALS AND METHODS

### Mice, tumor cell lines, cytokines and antibodies

Female BALB/c mice, 8 to 10 weeks old, were purchased from Nippon SLO (Sbizuoka, Japan). A murine HCC cell line, BNL, was kindly provided by Dr. S. Kuriyama (Nara Medical University, Nan., Japan). C26, a colon carcinoma cell line of BALB/c mouse, was provided from Tyugai Pharmaceutical Company, Tokyo. Murine recombinant IL-12 (mrIL-12) was kindly provided by Genetics Institute, Cambridge, MA. Human recombinant IL-2 (hrIL-2) was kindly provided by Sbionogi Pharmaceutical Company, Tokyo. Rat monoclonal antibodies against murine CD4, CD8, H-2K^{d} and I-A^{d}/I-E^{d} were purchased from Pharmingen, San Diego.

### Preparation of DCs

DCs were prepared with the method described by Inaba et al (Inaba et al., 1992, J. Exp. Med., 176:1693-1702) with modifications. Briefly, bone marrow cells were obtained from the femur and tibiae of female BALB/c mice (8 to 10 weeks old). Red blood cells were lysed by treatment With 0.83% ammonium chloride solution. The cells were incubated for 1 hour at 3700 on a plate coated with human γ-globulin (Cappel, Aurora, OH) (Yamaguchi et al., 1997, Stem Cell, 15:144-153). Nonadherent cells were harvested and cultured on 24-well plates (10⁵ cells/ml/well) in medium containing 10 ng/ml murine recombinant granulocyte/macrophage) colony-stimulating factor (GM-CSP) (Becton-Dickinson, Bedford, MA) and 60 U/mm of recombinant murine IL-4 (Becton-Dickinson). After 5 days of culture, nonadherent or loosely attached calls were collected by gentle pipetting and transferred to a 100-nun Petri dish. floating cells, which included many DCs, were collected after overnight culture. The cells obtained in this manner exhibited dendritic features and cell surface expression of MHC class 1, class II CD80, CD86, CD54 but not CD4, CD8 and CD4SR.

### Fusion of DCs and BNL cells

Fusion of DCs and BNL cells were performed according to Gong et al. (Gong et al., 1997, Nat. Med., 3:558-561) with modifications. Briefly, BNL cells were irradiated in the 35 Gy, mixed with DCs at a ratio of 1:3 (BNL:DC) and then centrifuged. Cell pellets were. treated with 50% polyethylene glycol (PEG 1460, Sigma Chemical Co., St. Louis, MO) for 1 minute at 370, after which the PEG solution was diluted with warm RPMI 1640 medium. The PEG treated cells were cultured overnight at 3700 in medium containing GM-CSF and IL-4.

### FACS analysis of the cells

To determine the efficiency of cell fusion, BNL cells were stained with PKH-26 (red fluorescence) and DCs were stained with PKH-2GL (green fluorescence). The cells stained with the fluorescent dyes were treated with PEG and cultured overnight as described above. The fusions were also stained with phycoerythin (PE) or fluorescein isothiocyanate (FITC) conjugated with monoclonal antibodies against I-A^{d}/I-E^{d}, CD80, CD86 and CD54 (Pharmingen, San Diego). Fluorescence profiles were generated with a FACSCalibur flow cytometer (Becton-Dickinson, San Jose, CA). Histograms and density plots were generated with the Cell Quest software package (Becton Dickinson, San Jose, CA).

### Scanning electron microscopy

Cells were fixed with 1.2% glutaraldehyde in 0.1 M phosphate buffer (pH 7.4). Fixed cells were attached to slides previously coated with 0.1 % poly-L-lysine, dehydrated in ascending concentrations of ethanol, treated with isoamyl acetate and critical-point dried with liquid CO₂. Specimens were coated with vacuum-evaporated, iron-sputtered gold and observed with a JSM-35 scanning electron microscope (Japan Electric Optical Laboratory, Tokyo, Japan) at an accelerating voltage of 10 kV.

### Injection of the fusions to mice and administration of IL-12

In tumor prevention studies, DC/BNL fusions were suspended in phosphate-buffered saline (PBS) and injected into the tail vein of mice (4 x 10⁵ cells/mouse), twice, at an interval of 2 weeks. One week after the second immunization, tumor challenge was performed by subcutaneous injection of 10⁶ BNL cells. The mice were monitored each week for the development of tumor by measurement of tumor size (≥ 3mm scored as positive). The control mice received phosphate-buffered saline (PBS), irradiated BNL cells (10⁵/mouse), DCs (3 x 10⁵/mouse) or mixture of irradiated BNL cells and DCs (4 x 10⁵/mouse, DC:BNL ratio 3:1) instead of the DC/BNL fusions, and were examined for development of the tumor as those which received the fusions. Each group consisted of 10 mice.

In treatment studies, the mice were divided into four groups. Ten mice in each group had BNL cells inoculated subcutaneously. In group A, DC/BNL fusions were injected subcutaneously on days 3 and 10 after inoculation of BNL cells. IL-12, dissolved in PBS containing 0.3% bovine serum albumin, was injected intraperitoneally on 2, 4 and 6 days after the first inoculation of the fusions and 3 and 5 days after the second inoculation. The mice in group B were treated in the same way as those in group A except that they did not receive IL-12. The mice in group C were treated in the same way as those in group A except that they did not receive the fusions. The mice in group D were treated in the same way as those in group A except that they received neither IL-12, nor the fusions.

### Assay of lytic activity of splenocytes against BNL cells

Splenocytes were obtained by gentle disruption of the spleen on a steel mesh and depletion of red blood cells by hypotonic treatment. Splenocytes from the mice were cultured in RPMI-1640 medium supplemented with 10% heat inactivated fetal calf serum (FCS) containing 50 U/ml of human recombinant IL-2 for 4 days. BNL cells (10⁴ cells/well) were labeled with ⁵¹Cr and incubated in RPMI-1640 medium supplemented with 10% heat inactivated FCS with splenocytes (effector cells) at the indicated effector target ratios in a volume of 200 ul in triplicate in a 96 multiwell plate for 4 hours at 37°C. After incubation, 100 µl of supernatant was collected and the percent specific ⁵¹Cr release was calculated with the following formula: percent ⁵¹Cr release =100 x (cpm experimental - cpm spontaneous release)\(cpm maximum release - cpm spontaneous release), where maximum release was that obtained from target cells incubated with 0.33N HCl and spontaneous release was that obtained from target cells incubated without the effector cells. For assessing inhibition of lytic activity by rat monoclonal antibodies against murine CD4, CD8, H-2K^{d}, I-A^{d}/I-E^{d}, 50 ug/ml of each antibody was added to the culture during the 4 hour incubation.

### Immunohistochemical studies

Immunofluorescent staining was performed by direct immunofluorescence. Frozen sections of tumor tissue were made and fixed with acetone for 10 minutes at room temperature. After washing with PBS, the sections were incubated in 10% normal goat serum in PBS for 20 minutes at room temperature, and then with the PE or FITC-labeled antibody in 10% normal goat serum in PBS for 2-3 hours at room temperature in a dark box. Sections were washed with PBS, mounted and observed under a fluorescent microscope.

### 7.2 RESULTS

### Characteristics of fusions of DCs and BNL cells

DCs and BNL cells were combined, treated with PEG and incubated overnight. Nonadherent and adherent cells obtained from PEG-treated cells exhibited dendritic features and epithelial characteristics, respectively, under a phase contrast microscope. Nonadherent cells expressed DC markers, I-A^{d} (MHC class II) and CD11c, by FACS analysis (data not shown). The finding that the adherent cells are negative for I-A^{d} and CD11c expression indicated that BNL cells were in the adherent cell fraction.

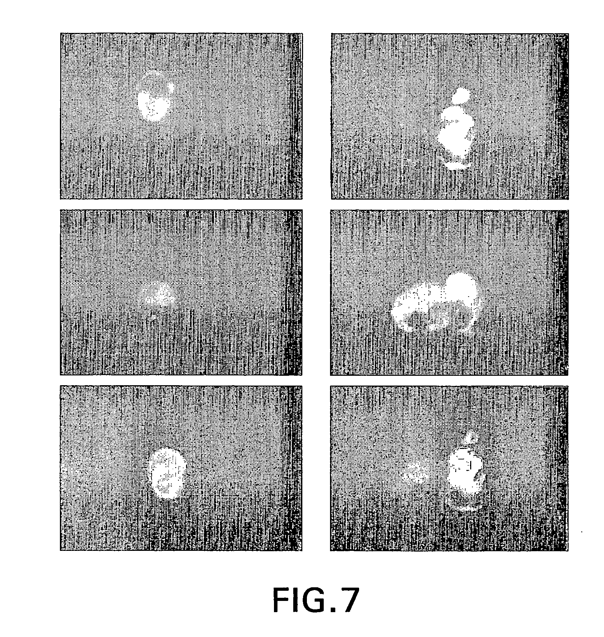

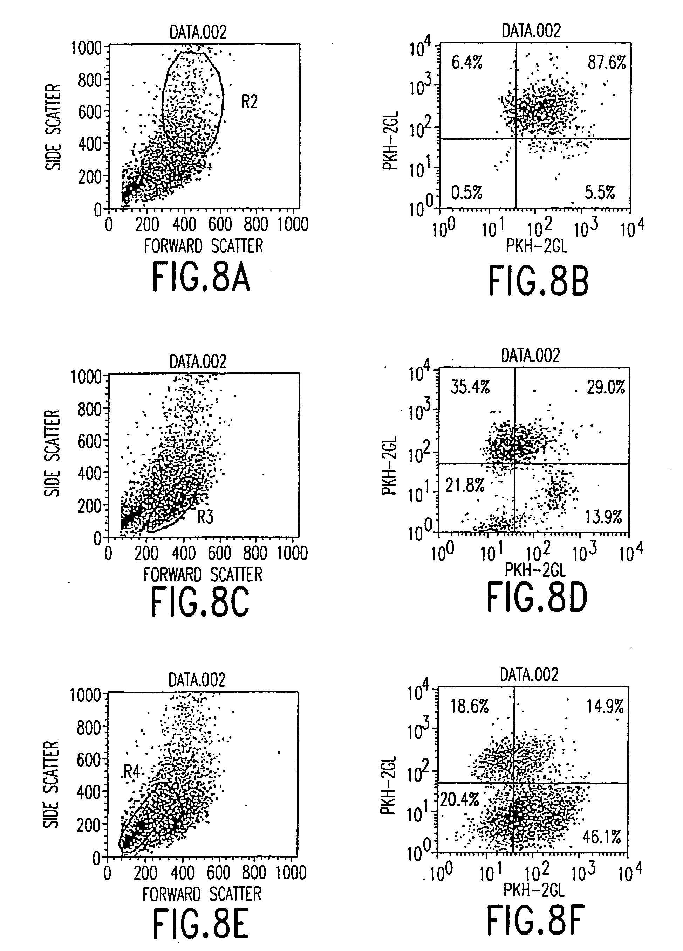

Prior to PEG treatment, DCs were treated with an FITC conjugated antibody against CD11c and BNL cells were stained with PKH-26. The cells were fused by PEG treatment and observed under a fluorescence microscope. Cells stained with both FITC (green) and PKH-26 (red) were observe among the PEG-treated cells (Figure 7). For determination of the fusion efficacy, DCs and BNL cells were stained with fluorescent dyes, PKH-2GL and PKH-26, respectively, and then treated with PEG. By FACS analysis, cells stained with both PKH-2GL and PKH-26, which were considered to be fusions of DCs and BNL cells, are shown in upper area of cell scattergram with high forward scatter and high side scatter (Figure 8). The cell fraction of high and moderate forward scatter and low side scatter contained many non-fused BNL cells, which those of low forward scatter and low side scatter contained non-fused DCs and non-fused BNL cells (Figure 8). About 30% of the nonadherent cells were fusions as judged from the width of area of double positive cells occupying in the whole scattergram.

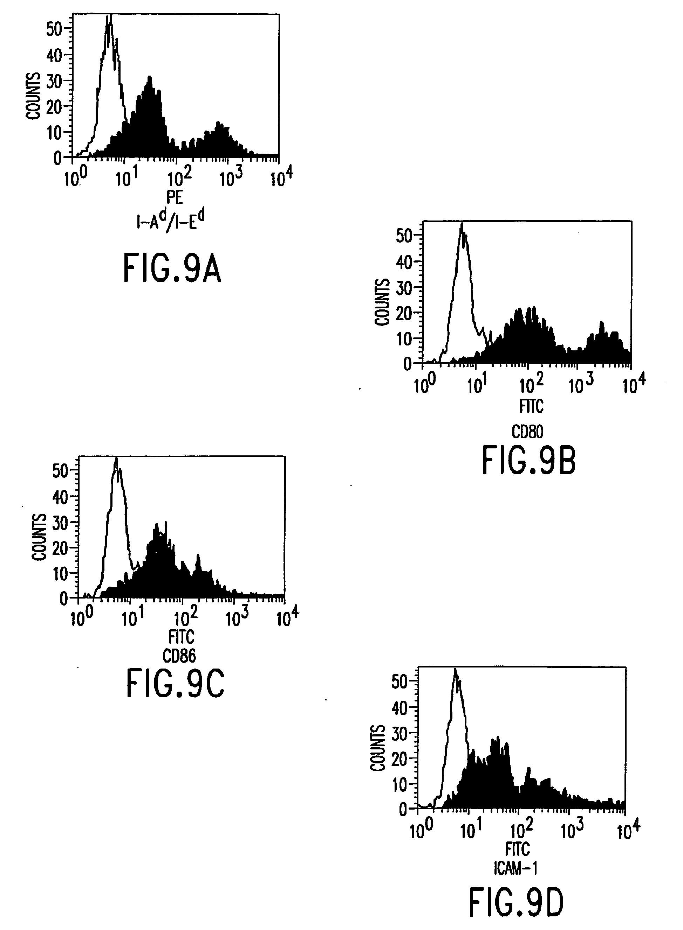

Phenotypes of the fusions were analyzed by FACS. The cell fraction positive for both PKH-2GL and PKH-26 were gated on scattergram and examined for antigen expression. I-A^{d}/I-E^{d} (MCH class II), CD80, CD86 and CD54 molecules, which are found on DCs, were expressed by the fusions (Figure 9).

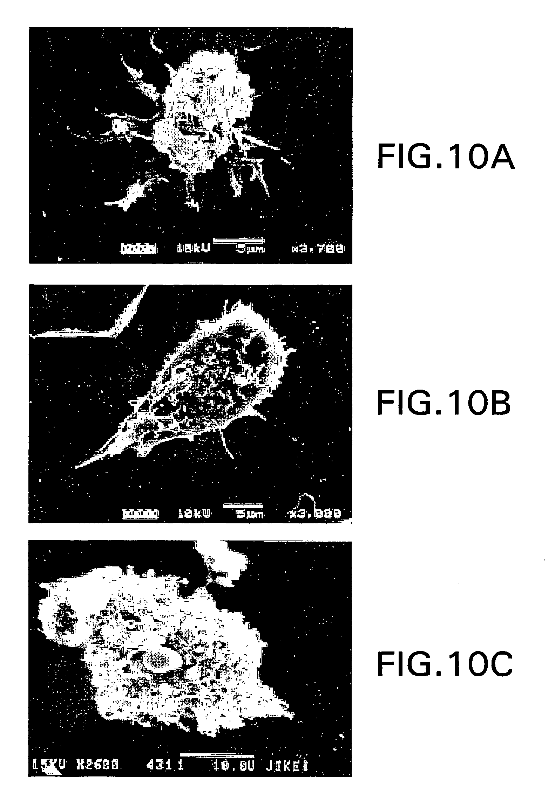

In addition, scanning electron microscopy showed that BNL cells express short processes on a plain cell surface, whereas DCs had many long dendritic processes. The nonadherent fusion cells were large and ovoid with short dendritic processes (Figure 10).

### Effect of vaccination with DC/BNL fusions on prevention of tumor development.

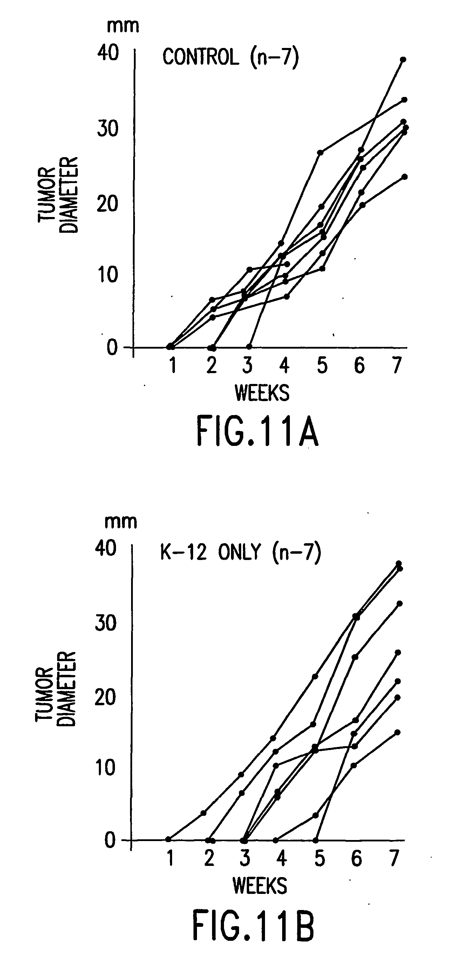

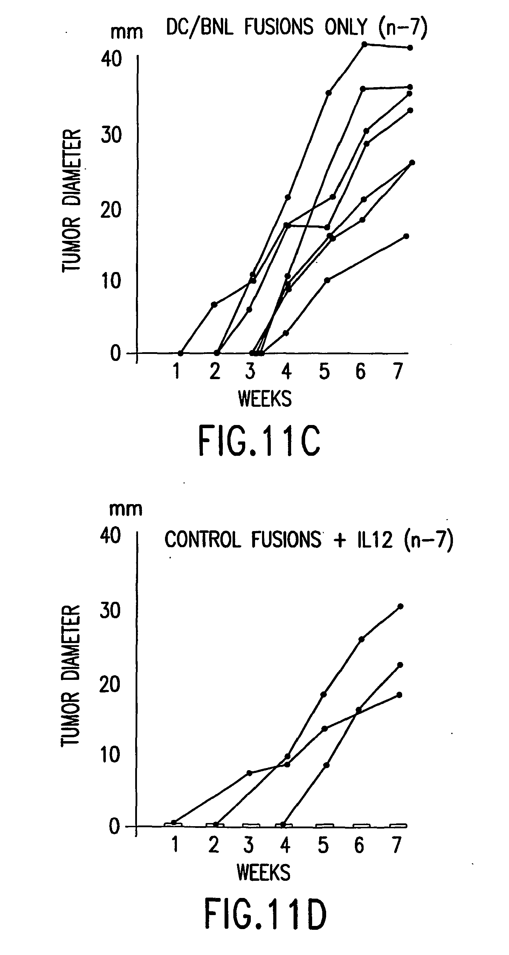

Vaccination with DC/BNL fusions resulted in the rejection of a challenge with BNL cells inoculated in BALB/c mice. By contrast, injection of only DCs or only irradiated BNL cells failed to prevent the development and growth of tumors (Figure 11). Injection of mixture of DCs and BNL cells, in numbers corresponding to those used to produce the fusions, transiently inhibited tumor growth, but after 4 weeks, tumors grew at rates comparable to controls. The finding that C26 colon carcinoma cells were not rejected by prior injection of DC/BNL fusions indicated that the immunity induced by DC/BNL fusions was specific for BNL cells (data not shown).

### Effects of vaccination with DC/BNL fusions on treatment of pre-established BNL tumors.

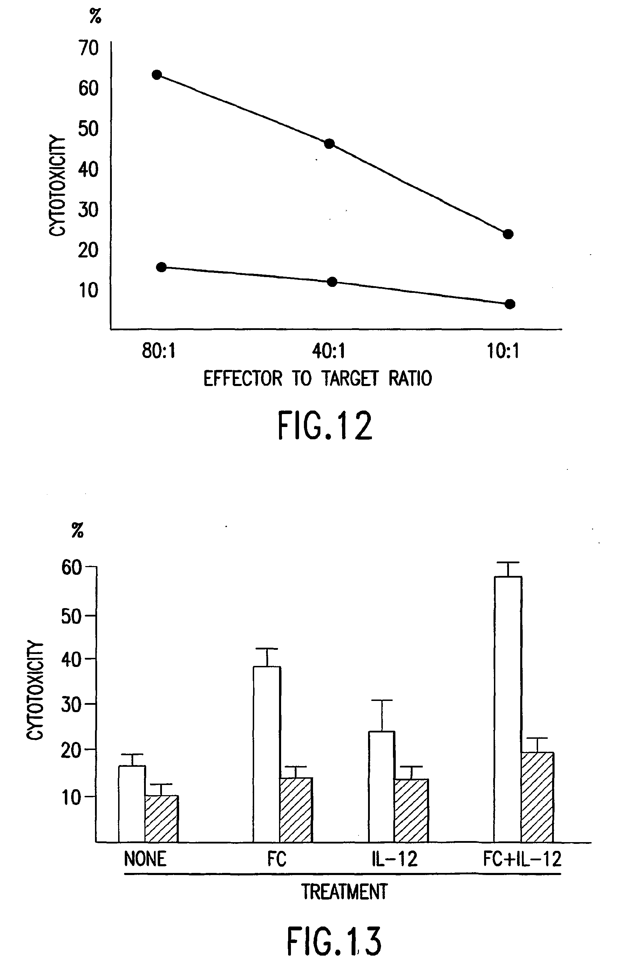

BNL cells (10⁶/mouse) were inoculated 3 days before treatment with DC/BNL fusions. The effect of treatment with DC/BNL fusion cells alone against BNL tumor was not significant (Figure 12). In addition, systemic administration of IL-12 (200 ng/mouse, intraperitoneal) alone had no significant therapeutic effect against growth of BNL cells; tumors were observed in all mice within 7 weeks after inoculation. However, injection of DC/BNL fusions followed by administration of IL-12 elicited a significant antitumor effect. Four of the seven mice showed no BNL tumor development. Thus, tumor incidence 7 weeks after BNL cell inoculation was 43% (3/7). Neither increasing nor decreasing the dose of IL-12 in this protocol improved the antitumor effect.

### Lytic activity of splenocytes against BNL cells in mice treated with DC/BNL fusions and IL-12.

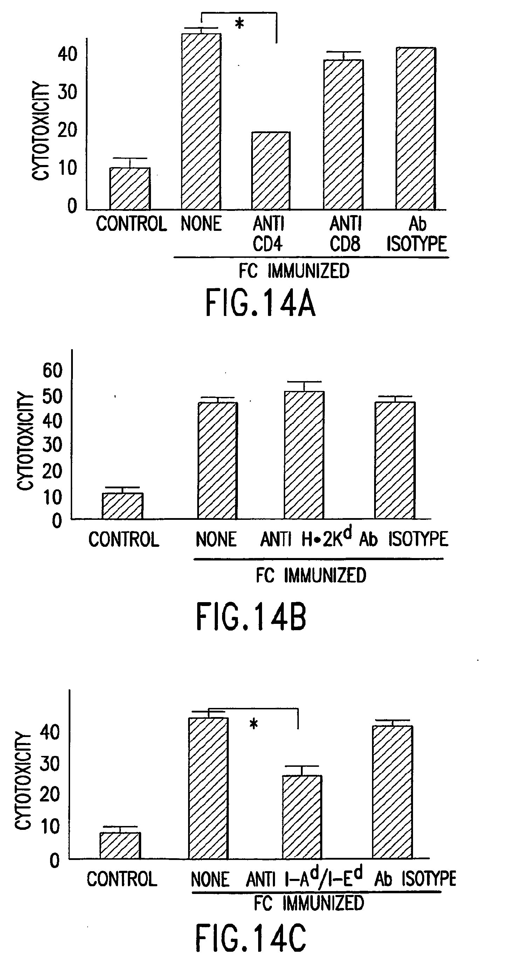

Significant cytolytic activity against BNL cells was observed using splenocytes derived from mice treated with DC/BNL fusions (Figure 13). Splenocytes from mice treated with both DC/BNL fusions and IL-12 showed stronger cytolytic activity against BNL cells than splenocytes from mice treated with DC/BNL fusions only. By contrast, there was no evidence of cytolytic activity against C26 colon carcinoma cells (Figure 14).

### Identification of effector cells induced by vaccination with the fusions

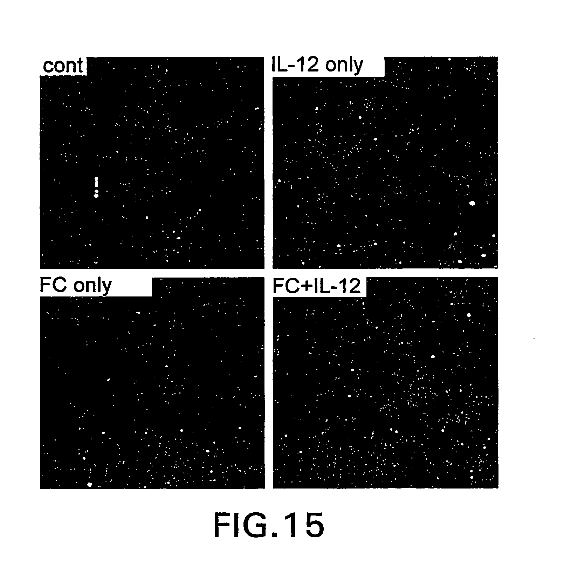

Splenocytes from mice immunized with DC/BNL fusions were examined for lytic activity against BNL cells in the presence of antibodies against CD4, CD8, H-2K^{d} and I-A^{d}/I-E^{d}. Lytic activity of the splenocytes treated with antibody against CD4 was significantly reduced, while those treated with antibody against CD8 exhibited almost the same lytic activity as those treated with an isotype identical antibody, rat IgG₂ₐ (Figure 15A). Lytic activity of the splenocytes from the fusion-treated mice was significantly inhibited when BNL cells were treated with antibody against I-A^{d}/I-E^{d}, but not H-2K^{d}. These results suggest that effector cells induced by immunization with DC/BNL fusions are CD4⁺ CTLs and the cytotoxicity is MHC class II-dependent.

### Immunohistochemical studies on BNL tumors growing in the fusion-treated mice.

BNL tumors which grew in spite of the prior injection of DC/BNL fusions were examined by immunohistochemistry, for infiltration of CD4⁺ cells and expression of I-A^{d}/I-E^{d} and for ICAM-1. In this study, DC/BNL fusions were injected subcutaneously, twice, at a two week interval. BNL cells, 10⁹ /mouse, were inoculated subcutaneously 7 days after the second injection of the fusions.

When small tumors emerged, some mice were treated with 200ng of IL-12 three times a week. The tumor was resected one day after the third administration of IL-12. CD4⁺ cells were detectable in the tumors that formed in the fusion-treated mice which had received IL-12. By contrast, few CD4⁺ cells were seen in tumors formed in mice treated with the fusions alone. I-A^{d}/I-E^{d} molecules were expressed more abundantly in BNL tumors formed in mice which had received administration of IL-12.

CD54 (Intercellular adhesion molecule 1; ICAM-1) was also expressed at higher levels on BNL tumor cells in mice treated with IL-12. These results suggest that main effector cells reactive with BNL cells induced by immunization with DC/BNL fusions were CD4⁺ CTLs. Moreover, treatment with IL-12 induces tumor cell susceptibility to CD4⁺ CTLs by enhanced expression of MHC class II and ICAM-1 molecules.

### 7.3. DISCUSSION

DCs are potent antigen-presenting cells that can present tumor antigens to naive T cells and prime them against these antigens (Grabbe et al., 1995, Immunolo. Today, 16:117-121; Shurin, M. R., 1996, Cancer Immunol., 43:158-164). A current focus of cancer immunotherapy is the utilization of DCs as an immunotherapeutic agent. Because DCs can process and present exogenous antigens to not only CD4⁺ T cells, but also CD8+ T cells, antitumor immunity induced by loading DCs with tumor lysate or antigenic peptides carried in the context of MHC molecules on the tumor cell surface may be a promising antitumor strategy (Paglia et al., 1996, J. Exp. Med., 183:317-322; Mayordomo et al., 1995, Nat. Med., 1:1297-1302; Celluzzi et al., 1996, J. Exp. Med., 183:283-287, Zivogel et al., 1996, J. Exp. Med., 183:87-97; Nestle et al., 1998, Nat. Med., 4:328-332; Porgador et al., 1995, J. Exp. Med., 182:255-260).

It has been reported that DCs fused with tumor cells induce antitumor immunity (Gong et al., 1997, Nat. Med. 3:558-561). In this setting, fusion cells present antigenic epitopes of tumor antigens to naive T cells and prime them against these antigens, because fusion cells simultaneously carry antigenic epitopes of the tumor cell and retain expression of MHC class I and class II molecules, co-stimulatory molecules (CD80, CD86) and intercellular adhesion molecule-1 (ICAM-1).

By fusing autologous DCs and tumor cells, obstacles to the induction of antitumor immunity such as MHC restriction, unique mutations of tumor antigens (Robbins et al., 1996, J. Exp. Med., 183:1185-1192; Brandle et al., 1996, J. Exp. Med., 183:2501-2508), and the multiplicity of tumor-specific epitopes may be overcome. Furthermore, problems of peptide-pulsed DCs, such as the low affinity of pulsed antigenic peptides to MHC molecules (Banchereau et al., 1998, Nature, 392:245-252) and the short life span of peptide-pulsed MHC class I molecules (Cella et al., 1997, Nature, 388:782-792) are not issues in fusion-based immunization. In addition, the number of BNL cells required for cell fusion is one half to one third that of DCs. A small number of requisite tumor cells is an advantage for the clinical application of fusion-based immunotherapy. Tumor cells that can be obtained at tumor biopsy might suffice as a source of fusion partners for DCs.

For the clinical application of DC/cancel cell fusions, assessment of the fusion efficacy of DCs and tumor cells by treatment with PEG and exclusion of cancer cells are important. Nonadherent cells showed DC markers, I-A^{d} and CD11c, whereas adherent cells did not, indicating that the nonadherent cell fraction contained fusion cells and DCs, and that most adherent cells were BNL cells which were not fused with DCs. In the nonadherent cell fraction, phase-contrast microscopy and scanning electron microscopy showed multi-dendritic cells larger than DCs. Two-color FACS analysis showed that approximately 30% of the PEG-treated nonadherent cells were positive for both PKH-2GL and PKH-26. Cells positive for both fluorescent dyes expressed MHC class II, CD80, CD86 and CD54 molecules which are required for antigen presentation. It is conceivable, therefore, that the fusions can present BNL tumor antigen(s) to naive T cells by means of DC capability. Immunization of BALB/c mice with DC/BNL was associated with protection against challenge with BNL cells. Moreover, splenocytes from the immunized mice showed significant lytic activity against BNL cells. By contrast, the finding that the splenocytes do not exhibit lytic activity against C26 murine colon carcinoma cells indicates that the antitumor immunity is specific for BNL cells. Mice immunized with a mixture of DCs and BNL cells, which were not treated with PEG, exhibited less protection against BNL cell challenge than did the DC/BNL fusion cells. Celluzzi, C.M. and Falo, LJ. (1998, J. Immunol, 160, 3081-5) found no difference of antitumor immunity between DC/B16 melanoma cell fusions and a mixture of DCs and B16 melanoma cells. This discrepancy might be due to differences in antigenicity between BNL HCC cells and B 16 melanoma cells.

IL-12 is a heterodimeric (p35/p40) cytokine originally termed cytotoxic lymphocyte maturation factor (CLMF) (Stem et al., 1990, Proc. Natl. Acad. Sci. USA, 87:6808-6812) or natural killer cell stimulating factor (NKSF) (Kobayashi et al., 1989, J. Exp. Med., 170:827-845). IL-12 plays a key role in differentiation of naive precursors to TH₁ cells to induce antitumor immunity (Tahara et al., 1995, Gene Ther., 2:96-106; Dustin et al., 1986, J. Immunol., 137:245-254; Schmitt et al., 1994, Eur. J. Immunol., 24:793-798). Dendritic cells that produce high levels of IL-12 drive naive helper T cells to differentiate to TH₁ (Macatonia et al., 1995, J. Immunol., 154:5071-5079). Splenocytes from mice treated with DC/BNL fusions in combination with IL-12 showed greater cytolytic activity against BNL cells than those treated with DC/BNL fusions alone (Figure 14). Helper T lymphocytes stimulated by a specific antigen and co-stimulated through CD80 and CD86 express IL-12 receptor (Igarashi et al., 1998, J. Immunol., 160:1638-1646). Immunization with DCs pulsed with tumor peptide and systemic administration of IL-12 elicit effective antitumor immunity (Zitvogel et al., 1996, Anal. New York Acad. Sci., 795:284-293). IFN-γ induced by IL-12 enhances the function of proteosomes and efficacy of antigen presentation by DCs (Griffin et al., 1998, J. Exp. Med., 187:97-104) and possibly by the fusion cells. In the present studies, systemic administration of IL-12 alone had no effect against pre-established BNL tumors. Nonspecific activation of CTLs or NK cells by treatment with IL-12 is apparently not sufficient to induce tumoricidal activity. The present studies also demonstrate that induction of specific CTLs by immunization with DC/tumor cell fusions and activation of the induced CTLs by IL-12 produce effective and tumor-specific antitumor immunity. It is also conceivable that DC-tumor cell fusions can not produce sufficient IL-12 to induce Th1 condition. IL-12 produced and released from DCs presenting a specific antigen to naive T helper cells activates Th1 cells (Macatonia et al., 1995, J. Immunol., 154:5071-5079). If the ability of DC to produce IL-12 is attenuated by cell fusion, systemic administration of IL-12 to the fusion-immunized host may contribute to the development of Th1 cells and generation of specific CTLs. Another possibility is that antigen presentation by the fusions induces a Th2 response and secretion of IL-10, an inhibitor of IL-12 production (Hino et al., 1996, Eur. J. Immunol., 26:623-628). Systemic administration of IL-12 could also inhibit Th2 response and generate tumoricidal CTLs.

Cytolytic activity of splenocytes from mice treated with the fusions was inhibited by treatment of the splenocytes with antibody against CD4 and treatment of the target cells with antibody against I-A^{d}/I-E^{d}. These findings suggest that BNL-specific effector cells are CD4⁺ CTLs and cytotoxicity is dependant on MHC class II (Shinohara N.,1987, Cellular Immunol., 107:395-407; Ozdemirli et al., 1992, J. Immunol., 149:1889-1885; Yasukawa et al., 1993, Blood, 81:1527-1534). DCs present specific tumor antigen to CD8⁺ CTLs and tumoricidal activity is MHC class I dependent (Porgador et al., 1995, J. Exp. Med., 182:255-260). Although CD4⁺ CTLs are uncommon, CD4⁺ CTLs work in almost the same manner as CD8⁺ CTLs (Yasukawa et al., 1993, Blood, 81:1527-1534). In this study, cytolytic activity was not inhibited by treatment of effector cells with antibodies against CD8 nor treatment of the target cells with antibody against MHC class I. Expression of MHC class II (I-A^{d}/I-E^{d}) molecules on BNL tumor in vivo was greatly enhanced when BNL bearing mice were treated with IL-12. This response may be due to the induction of interferon-γ, tumor necrosis factor (TNF) or interleukin-1 (Gately et al., 1994, Int. Immunol., 6:157-167; Nastala et al., 1994, J. Immunol., 153:1697-1706). Enhanced expression of MHC class II molecules increases exposure of antigenic peptides from BNL tumor antigens to CD4⁺ CTLs. Furthermore, expression of ICAM-1 by BNL tumor tissue was more enhanced by treatment of the tumor-bearing mice with IL-12. This effect could also be due to the effect of IFN-γ or IL-1 directly or indirectly induced by IL-12 (Dustin et al., 1986, J. Immunol., 137:245-254). These results suggest that CTLs are able to attach to endothelial cells of the tumor and migrate into the tumor tissue more efficiently by IL-12 treatment, leading to enhanced antitumor activity against established lesions.

The development and frequent recurrence of multicentric HCC are serious problems in patients with virus-induced cirrhosis. Therefor, methods of preventing the development of HCC are needed. Small HCCs can be detected with ultrasonography and curatively treated with percutaneous ethanol injection therapy or surgical resection. To prevent the development of new HCCs and treat remaining micrometastases, tumor cells obtained at biopsy or resection can be fused with DCs. Thus, as demonstrated in this example, immunization with fusions of autologous DCs and tumor cells combined with IL-12 administration is a promising method for the treatment of HCC.

## Claims

1. Use of a therapeutically effective amount of a fusion cell or a composition comprising fusion cells, wherein said fusion cells are formed by the fusion of an autologous non-dendritic tumor cell and a dendritic cell which has the same class I MHC haplotype as a mammal to be treated in combination with a IL-12 which stimulates a cytotoxic T cell response for the manufacture of a medicament for treating or preventing cancer.

2. Use according to claim 1 wherein the dendritic cells are terminally differentiated and express the cell surface marker CD83.

3. Use of claim 1 or 2, wherein the dendritic cell is obtained from human blood monocytes.

4. Use of claim 1, or 2 wherein the non-dendritic cell is a tumor cell obtained from the mammal.

5. Use of claim 1 or 2, wherein the non-dendritic cell is obtained from a tumor cell line derived from a tumor cell obtained from the mammal in which the fusion cell is to be administered.

6. Use of claim 1 or 2, wherein the mammal is a human.

7. Use of claim 1 - 3, wherein the mammal is selected from the group consisting of a cow, a horse, a sheep, a pig, a fowl, a goat, a cat, a dog, a hamster, a mouse and a rat.

8. Use of claim 1 or 2, wherein the cancer is selected from the group consisting of renal cell carcinoma, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, leukemias, acute lymphocytic leukemia, acute myelocytic leukemia; chronic leukemia, polycythemia vera, lymphoma, multiple myeloma, Waldenström's macroglobulinemia, and heavy chain disease.

9. A pharmaceutical composition comprising a fusion cell comprising a dendritic cell fused to a non-dendritic cell, which non-dendritic cell is freshly isolated or obtained from a primary tumor cell culture and IL-12 which stimulates a cytotoxic T cell response.

10. A pharmaceutical composition according to claim 9 wherein the dendritic cells are terminally differentiated and express the cell surface marker CD83.

11. The pharmaceutical composition of claim 9 or 10 wherein the dendritic cell and the non-dendritic cell are human.

## Patentansprüche

1. Verwendung einer therapeutisch wirksamen Menge einer Fusionszelle oder einer Zusammensetzung, die Fusionszellen aufweist, wobei die Fusionszellen durch die Fusion einer autologen nichtdendritischen Tumorzelle und einer dendritischen Zelle gebildet werden, die den gleichen MHC Klasse I-Haplotyp aufweist wie ein zu behandelndes Säugetier, in Kombination mit einem IL-12 (Interleukin-12), das eine zytotoxische T-Zellen-Antwort stimuliert, für die Herstellung eines Medikaments zur Behandlung oder Verhütung von Krebs.

2. Verwendung nach Anspruch 1, wobei die dendritischen Zellen terminal differenziert sind und den Zelloberflächennmarker CD83 exprimieren.

3. Verwendung nach Anspruch 1 oder 2, wobei die dendritische Zelle aus Humanblut-Monozyten gewonnen wird.

4. Verwendung nach Anspruch 1 oder 2, wobei die nichtdendritische Zelle eine aus dem Säugetier gewonnene Tumorzelle ist.

5. Verwendung nach Anspruch 1 oder 2, wobei die nichtdendritische Zelle aus einer Tumorzellinie gewonnen wird, die von einer Tumorzelle abgeleitet wird, die man von dem Säugetier gewinnt, dem die Fusionszelle verabreicht werden soll.

6. Verwendung nach Anspruch 1 oder 2, wobei das Säugetier ein Mensch ist.

7. Verwendung nach Anspruch 1 - 3, wobei das Säugetier aus der Gruppe ausgewählt ist, die aus einem Rind, einem Pferd, einem Schaf, einem Schwein, einem Huhn, einer Ziege, einer Katze, einem Hund, einem Hamster, einer Maus und einer Ratte besteht.

8. Verwendung nach Anspruch 1 oder 2, wobei das Karzinom aus der Gruppe ausgewählt ist, die aus Nierenzellkarzinom, Fibrasarkom, Myxosarkom, Liposarkom, Chondrosarkom, Osteosarkom, Chordom, Angiosarkom, Endotheliosarkom, Lymphangiosarkom, Lymphangioendotheliosarkom, Synoviom, Mesotheliom, Ewing-Tumor, Leiomyosarkom, Rhabdomyosarkom, Kolonkarzinom, Pankreaskarzinom, Brustkrebs, Ovarialkarzinom, Prostatakarzinom, Plattenepithelkarzinom, Basalzellenkarzinom, Adenokarzinom, Schweißdrüsenkarzinom, Talgdrüsenkarzinom, papillärem Karzinom, papillärem Adenokarzinom, Cystadenokarzinom, medullärem Karzinom, Bronchialkarzinom, Hepatom, Gallengangkarzinom, Choriokarzinom, Seminom, Embryonalkarzinom, Wilms-Tumor, Zervikalkarzinom, Hodenkrebs, Lungenkarzinom, kleinzelligem Lungenkarzinom, Blasenkarzinom, Epithelkarzinom, Gliom, Astrozytom, Medulloblastom, Kraniopharyngiom, Ependymom, Pinealom, Hämangioblastom, Akustikusneurinom, Oligodendrogliom, Meningiom, Melanom, Neuroblastom, Retinoblastom, Leukämien, akuter lymphozytischer Leukämie, akuter myelozytischer Leukämie, chronischer Leukämie, Polyzytämia vera, Lymphom, multiplem Myelom, Waldenströmscher Makroglobulinämie und Schwerkettenkrankheit besteht.

9. Pharmazeutische Zusammensetzung mit einer Fusionszelle, die eine mit einer nichtdendritischen Zelle fusionierte dendritische Zelle aufweist, wobei die nichtdendritische Zelle aus einer primären Tumorzellkultur frisch isoliert oder gewonnen wird, und IL-12 aufweist, das eine zytotoxische T-Zellen-Antwort stimuliert.

10. Pharmazeutische Zusammtensetzung nach Anspruch 9, wobei die dendritischen Zellen terminal differenziert sind und den Zelloberflächenmarker CD83 exprimieren.

11. Pharmazeutische Zusammensetzung nach Anspruch 9 oder 10, wobei die dendritische Zelle und die nichtdendritische Zelle Humanzellen sind.

## Revendications

1. Utilisation d'une quantité thérapeutiquement efficace d'une cellule de fusion ou d'une composition comprenant des cellules de fusion, dans laquelle lesdites cellules de fusion sont formées par la fusion d'une cellule tumorale non dendritique autologue et d'une cellule dendritique qui a le même haplotype du CMH de classe 1 qu'un mammifère devant être traité, en combinaison avec une IL-12 qui stimule une réponse des lymphocytes T cytotoxiques, pour la fabrication d'un médicament pour traiter ou prévenir un cancer.

2. Utilisation selon la revendication 1, dans laquelle les cellules dendritiques sont différentiées terminalement et expriment le marqueur de surface cellulaire CD83.

3. Utilisation selon la revendication 1 ou 2, dans laquelle la cellule dendritique est obtenue à partir de monocytes de sang humain.

4. Utilisation selon la revendication 1 ou 2, dans laquelle la cellule non dendritique est une cellule tumorale obtenue auprès du mammifère.

5. Utilisation selon la revendication 1 ou 2, dans laquelle la cellule non dendritique est obtenue à partir d'une lignée de cellules tumorales issue d'une cellule tumorale obtenue auprès du mammifère dans lequel la cellule de fusion doit être administrée.

6. Utilisation selon la revendication 1 ou 2, dans laquelle le mammifère est un humain.

7. Utilisation selon les revendications 1-3, dans laquelle le mammifère est choisi dans le groupe constitué par une vache, un cheval, un mouton, un cochon, une volaille, une chèvre, un chat, un chien, un hamster, une souris et un rat.

8. Utilisation selon la revendication 1 ou 2, dans laquelle le cancer est choisi dans le groupe constitué par un carcinome à cellules rénales, un fibrosarcome, un myxosarcome, un liposarcome, un chondrosarcome, un sarcome ostéogénique, un chordome, un angiosarcome, un endothéliosarcome, un lymphangiosarcome, un lymphangioendothéliosarcome, un synoviome, un mésothéliome, une tumeur d'Ewing, un léiomyosarcome, un rhabdomyosarcome, un carcinome du côlon, un cancer pancréatique, un cancer du sein, un cancer de l'ovaire, un cancer de la prostate, un carcinome à cellules squameuses, un carcinome à cellules basales, un adénocarcinome, un carcinome sudoripare, un carcinome sébacé, un carcinome papillaire, des adénocarcinomes papillaires, un cystadénocarcinome, un carcinome médullaire, un carcinome bronchogénique, un hépatome, un carcinome biliaire, un choriocarcinome, un séminome, un carcinome embryonnaire, une tumeur de Wilm, un cancer du col de l'utérus, une tumeur testiculaire, un carcinome du poumon, un carcinome du poumon à petites cellules, un carcinome de la vessie, un carcinome épithélial, un gliome, un astrocytome, un médulloblastome, un craniopharyngiome, un épendymome, un pinéalome, un hémangioblastome, un neurinome acoustique, un oligodendrogliome, un méningiome, un mélanome, un neuroblastome, un rétinoblastome, des leucémies, une leucémie lymphoïde aiguë, une leucémie myéloïde aiguë; une leucémie chronique, une polycythémie vraie, un lymphome, un myélome multiple, une macroglobulinémie de Waldenström, et une maladie des chaînes lourdes.

9. Composition pharmaceutique comprenant une cellule de fusion comprenant une cellule dendritique fusionnée à une cellule non dendritique, laquelle cellule non dendritique est fraîchement isolée ou obtenue à partir d'une culture de cellules tumorales primaires, et IL-12 qui stimule une réponse des lymphocytes T cytotoxiques.

10. Composition pharmaceutique selon la revendication 9, dans laquelle les cellules dendritiques sont différenciées terminalement et expriment le marqueur de surface cellulaire CD83.

11. Composition pharmaceutique selon la revendication 9 ou 10, dans laquelle la cellule dendritique et la cellule non dendritique sont humaines.
